# EUROPÄISCHE PATENTSCHRIFT

(11) **EP 1 558 593 B1**
(45) Veröffentlichungstag und Bekanntmachung des Hinweises auf die Patenterteilung: **03.03.2010**
(21) Anmeldenummer: 03779830.3
(22) Anmeldetag: 03.11.2003
(51) Int. Cl.: C07D 277/82, C07D 277/42, C07F 7/00, B01J 31/00, C08F 10/00, C08F 4/64, C08F 4/62

(54) **ÜBERGANGSMETALLKATALYSATOREN FÜR (CO)POLYMERISATION VON OLEFINISCHEN MONOMEREN**
TRANSITION METAL CATALYSTS FOR (CO)POLYMERIZING OLEFINIC MONOMERS
CATALYSEURS A METAL DE TRANSITION POUR LA (CO)POLYMERISATION DE MONOMERES OLEFINIQUES

(30) Priorität: 04.11.2002 DE 10251513
(43) Veröffentlichungstag der Anmeldung: 03.08.2005
(73) Patentinhaber: Basell Polyolefine GmbH, 50389 Wesseling (DE)
(72) Erfinder: PREISHUBER-PFLÜGL, Peter, 67061 Ludwigshafen (DE); OKUDA, Jun, 55218 Ingelheim (DE); REIMER, Valentine, 64625 Bensheim (DE); KRISTEN, Marc Oliver, 65779 Kelkheim (DE)
(86) Internationale Anmeldenummer: PCT/EP2003/012200
(87) Internationale Veröffentlichungsnummer: WO 2004/041796

(56) Entgegenhaltungen:
- EP-A- 0 874 005
- EP-A- 0 950 667
- EP-A- 1 174 442
- WO-A-00/50470
- SHARMA,R.C.: "SYNTHESIS AND STRUCTURAL STUDIES OF CO(II),NI(II),ZN(II9 AND CD(II9 METAL COMPLEXES OF 2-HYDROXY-5-METHYL BENZENE-1,3-BIS-(CARBALIDINE-2-AMINO THIAZOLE)" ASIAN JOURNAL OF CHEMISTRY, Bd. 14, Nr. 1, 2002, Seiten 503-5, XP008027868
- SHARMA,R.C.: "STUDIES ON CO(II) AND NI(II) COMPLEXES OF NEWLY SYNTHESISED HETEROCYCLIC SCHIFF BASES." JOURNAL OF THE INSTITUTION OF CHEMISTS, Bd. 74, Nr. 6, 2002, Seiten 188-190, XP008027869 INDIA

## Beschreibung

Die vorliegende Erfindung betrifft Metallkomplexe, die aus einem Übergangsmetall und mindestens einem mehrzähnigen Liganden aufgebaut sind, ein Verfahren zur Herstellung der Metallkomplexe, die mehrzähnigen Liganden selbst sowie ihre Herstellung, katalytisch aktive Zusammensetzungen, enthaltend den erfindungsgemäßen Metallkomplex, die Verwendung der erfindungsgemäßen katalytischen Zusammensetzung zur Polymerisation oder Copolymerisation von Olefinen sowie ein Verfahren zur Polymerisation oder Copolymerisation von Olefinen, worin die erfindungsgemäße katalytische Zusammensetzung eingesetzt wird und ein Polymer oder ein Copolymer herstellbar nach dem erfindungsgemäßen Verfahren.

Polymere und Copolymere von Olefinen sind wirtschaftlich von großer Bedeutung, da die Monomere in großen Mengen leicht zugänglich sind und weil sich die Polymere durch Variation des Herstellungsverfahrens oder der Verarbeitungsparameter in weiten Bereichen variieren lassen. Besondere Aufmerksamkeit bei dem Herstellverfahren der Polymere oder Copolymere gilt dabei dem verwendeten Katalysator. Neben Ziegler-Natta-Katalysatoren gewinnen verschiedene Single-Site-Katalysatoren an Bedeutung, wobei als Zentralatome Übergangsmetalle wie Zr (z.B. in Metallocenkatalysatoren), Ni, Pd oder Fe oder Co eingesetzt werden.

Die gut untersuchten Metallocenkatalysatoren haben für den großtechnischen Einsatz Nachteile. Die am häufigsten verwendeten Metallocene sind Zirkonocene und Hafnocene, die sehr hydrolyseempfindlich sind. Des weiteren sind die meisten Metallocene empfindlich gegenüber einer Vielzahl von Katalysatorgiften wie Alkohole, Ether oder Kohlenmonoxid, was eine sorgfältige Reinigung der als Monomere eingesetzten Olefine erfordert.

EP-A 0 874 005 betrifft Polymerisationskatalysatoren, die Übergangsmetallverbindungen enthalten, die einen oder mehrere bidentate (zweizähnige) Liganden aufweisen. Bei den Übergangsmetallkomplexen handelt es sich bevorzugt um Ti-Komplexe, die Salicylaldiminliganden aufweisen. Dabei kann das Aldimin-Stickstoffatom Phenylsubstituenten tragen oder in einen 6-gliedrigen Ring eingebaut sein. Mit diesen Katalysatoren werden in der Regel niedermolekulare Polyethylene erzeugt, die als Werkstoffe wenig geeignet sind.

EP-A 0 950 667 betrifft ebenfalls Polymerisationskatalysatoren, die ÜbergangsmetallVerbindungen enthalten, die einen oder mehrere bidentate (zweizähnige) Liganden aufweisen. Dabei erfolgt die Koordination zum Übergangsmetall durch mindestens ein Stickstoffatom und mindestens ein an einen aromatischen Rest gebundenes Atom. Als Übergangsmetalle werden gemäß den Beispielen bevorzugt Titan und Zirkon eingesetzt.

Richard F. Jordan et al. Organometallics 1997, 16, 3282 bis 3302 betrifft Übergangsmetallkomplexe - von Gruppe IV Übergangsmetallen, die zweizähnige 8-Chinolinolato-Liganden aufweisen. Diese Metallkomplexe werden in der Ethylenpolymerisation eingesetzt. Die Aktivitäten der Komplexe sind jedoch sehr gering.

WO 98/27124 betrifft Eisen- und Cobalt-Komplexe von 2,6-Pyridincarboxaldehyd-bis(iminen) und 2-6-Diacylpyridin-bis(iminen), die als Katalysatoren zur Ethylenpolymerisation eingesetzt werden. Bei diesen Katalysatoren handelt es sich um dreizähnige Liganden, wobei das Übergangsmetall über drei Stickstoffatome des Liganden koordiniert ist.

Aufgrund der großen kommerziellen Bedeutung von Polyolefinen ist die Suche nach möglichst vielseitigen polymerisationsaktiven Übergangsmetallkomplexen auch weiterhin von großer Bedeutung. Dabei ist, neben einer hohen Aktivität der Übergangsmetallkatalysatoren, deren Stabilität ebenfalls von besonderer Wichtigkeit. Viele monodentate (einzähnige) oder bidentate (zweizähnige) Liganden ergeben mit verschiedenen Übergangsmetallsalzen aktive Polymerisationskatalysatoren. Diese Katalysatoren zeigen jedoch häufig eine Abnahme der Konzentration der aktiven Spezies im Verlaufe der Polymerisation.

Aufgabe der vorliegenden Erfindung ist es daher, Liganden, diese enthaltende Übergangsmetallkomplexe sowie diese Übergangsmetallkomplexe enthaltende Polymerisationskatalysatoren bereitzustellen, die stabilisiert sind und somit das Problem der Abnahme der Konzentration der aktiven Spezies im Verlaufe der Polymerisation lösen.

Diese Aufgabe wird gelöst durch eine Verbindung der allgemeinen Formel (Ia) oder (Ib) 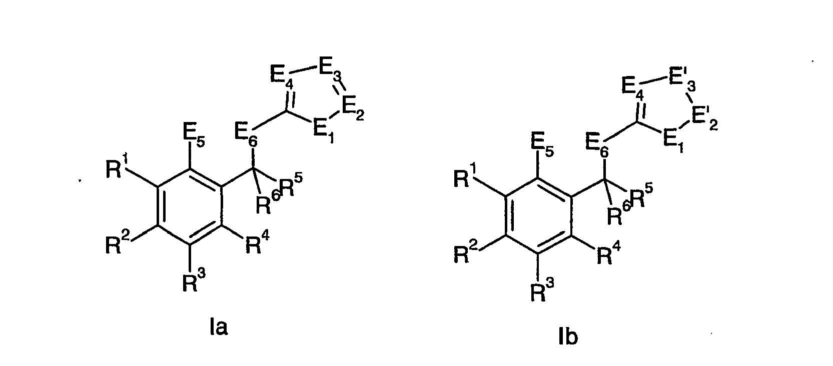 worin
in Formel (Ia)
- E1: O, S, Se, Te, NR, CR₂, PR, bevorzugt O oder S, besonders bevorzugt S
- E2, E3: CR, N, P, bevorzugt CR,
- E4: N, P, bevorzugt N,
- E5: OH, SH, NHR, bevorzugt OH oder OR', SR', NRR',
- E6: NH, PH, bevorzugt NH oder NR', PR',
- R⁵, R⁶: Wasserstoff oder ein linearer, verzweigter oder cyclischer Alkylrest oder ein Arylrest,
- R¹, R², R³ R⁴: Wasserstoff ein linearer, verzweigter oder cyclischer Alkylrest, ein Arylrest, Halogen oder eine Nitrogruppe,
- R: Wasserstoff, ein linearer, verzweigter oder cyclischer Alkylrest oder ein Arylrest,
- R': ein linearer, verzweigter oder cyclischer Alkylrest oder ein Arylrest,
bedeuten, wobei mindestens eine der Gruppen E5 oder E6 ein Wasserstoffatom enthält,
wobei bevorzugt sowohl E5 als auch E6 jeweils ein Wasserstoffatom enthalten; und in Formel (Ib)
die Symbole E1, E4, E5, E6, R⁵, R⁶, R¹, R², R³, R⁴, R und R¹ dieselben Bedeutungen aufweisen wie in Formel (Ia)
und
E2' und E3' O, S, Se, Te, NR, CR₂, PR, bevorzugt CR₂ bedeuten.

Bevorzugt ist in den erfindungsgemäßen Verbindungen gemäß Formel Ia oder Ib E1 S oder O, besonders bevorzugt S, und E4 N. Ganz besonders bevorzugt ist E1 S oder O, bevorzugt S, E4 N und E6 NH.

Diese Verbindungen sind hervorragend als Liganden in Übergangsmetallkomplexen geeignet. Die erfindungsgemäßen Verbindungen weisen mehrere Koordinationsstellen auf. So können durch zusätzliche Donor-Akzeptor-Wechselwirkungen die Übergangsmetallzentren von Übergangsmetallkomplexen stabilisiert werden. Diese Übergangsmetallkomplexe sind dann hervorragend zum Einsatz in der Polymerisation von Olefinen geeignet.

Durch die Auswahl der Substituenten (elektronenschiebend oder -ziehend) an dem durch E1, E2, E3, E4 und ein weiteres Kohlenstoffatom gebildeten heterozyklischen 5-Ring kann die Nukleophilie des Metallzentrums eines die erfindungsgemäßen Verbindungen der allgemeinen Formel Ia oder Ib enthaltenden Übergangsmetallkomplexes eingestellt werden. Bei Einsatz dieser Übergangsmetallkomplexe in der Polymerisation von Polyolefinen können dadurch die Liganden an die jeweils eingesetzten Übergangsmetallatome bzw. die in der Polymerisation eingesetzten Monomere angepasst werden.

Geeignete lineare oder verzweigte Alkylreste sind Alkylreste mit 1 bis 30 Kohlenstoffatomen, bevorzugt 1 bis 20 Kohlenstoffatomen, wie Methyl, Ethyl, n-Propyl, Iospropyl, n-Butyl, Isobutyl, sec-Butyl, tert-Butyl, neo-Pentyl und n-Hexyl.

Geeignete cyclische Alkylreste sind cyclische gesättigte Kohlenwasserstoffreste mit 3 bis 30 Kohlenstoffatomen, bevorzugt 3 bis 20 Kohlenstoffatomen wie Cyclopropyl, Cyclobutyl, Cyclopentyl, Cyclohexyl und Adamantyl.

Geeignete Arylreste sind solche mit 6 bis 30 Kohlenstoffatomen, bevorzugt 6 bis 20 Kohlenstoffatomen wie Phenyl, Benzyl, Naphthyl, Biphenyl, Terphenyl, Phenantryl und Anthracenyl. Die Arylreste können des weiteren alkylsubstituiert sein. Geeignete alkylsubstituierte Arylreste sind Tolyl, Isopropylphenyl, t-Butylphenyl, Dimethylphenyl und Di-t-Butylphenyl.

In den oben genannten Alkyl- und Arylresten können einzelne Wasserstoffatome durch Halogenatome ersetzt sein. Beispiele für solche halogenierten Alkyl- und Arylreste sind Trifluoromethyl, Pentafluorophenyl und Chlorophenyl.

Es ist des weiteren möglich, dass in den Alkyl- und Arylresten ein oder mehrere Wasserstoffatome durch andere Kohlenwasserstoffreste ersetzt sind, zum Beispiel arylsubstituierte Alkylreste wie Benzyl und Cumyl.

Besonders bevorzugt sind R¹, R², R³ und R⁴ Wasserstoff, Halogen, eine Nitrogruppe oder lineare oder verzweigte Alkylreste mit 1 bis 20 Kohlenstoffatomen wie Methyl, Ethyl, n-Propyl, Isopropyl, n-Butyl, Isobutyl, sec-Butyl, t-Butyl, neo-Pentyl und n-Hexyl, ganz besonders bevorzugt mit 1 bis 4 Kohlenstoffatomen wie Methyl, Ethyl, n-Propyl, Isopropyl, n-Butyl, Isobutyl, sec-Butyl und t-Butyl. Des weiteren bevorzugt sind R¹, R², R³ und R⁴ Arylreste mit 6 bis 20 Kohlenstoffatomen wie Phenyl, Naphthyl, Biphenyl, Terphenyl, Phenanthryl und Anthracenyl, ganz besonders bevorzugt sind Phenyl und Naphthyl, sowie substituierte Arylreste wie Tolyl und Cumyl.

Ganz besonders bevorzugt ist mindestens einer der Reste R¹, R², R³ und R⁴ ein Wasserstoffatom, insbesondere bevorzugt sind zwei der genannten Reste ein Wasserstoffatom. Die übrigen Reste sind bevorzugt Alkyl- und/oder Arylreste, besonders bevorzugt Alkylreste, wie vorstehend erwähnt.

Entsprechende Definitionen wie für R¹, R², R³ und R⁴ gelten ebenfalls für die Reste R⁵ und R⁶ sowie R. Die vorstehend genannten Definitionen für bevorzugt eingesetzte Alkyl- und Arylreste gelten ebenfalls für den Rest R¹.

Bevorzugte Definitionen für E1, E2, E2', E3', E4, E5 und E6 sind ebenfalls bereits vorstehend genannt. Besonders bevorzugt sind Verbindungen der allgemeinen Formel Ia oder Ib, in denen E1 S oder O, bevorzugt S, ist und gleichzeitig E4 N ist. Besonders bevorzugt sind E2 und E3 in Verbindungen der Formel Ia CR, wobei bevorzugte Definitionen für R bereits vorstehend genannt sind. In Verbindung Ib sind E2' und E3' besonders bevorzugt CR₂, wobei bevorzugte Definitionen für R ebenfalls vorstehend genannt sind und die beiden an das Kohlenstoffatom in E2' bzw. E3' gebundenen Reste R gleich oder verschieden sein können.

Ganz besonders bevorzugt sind Verbindungen Ia und Ib gemäß den folgenden Strukturformeln, 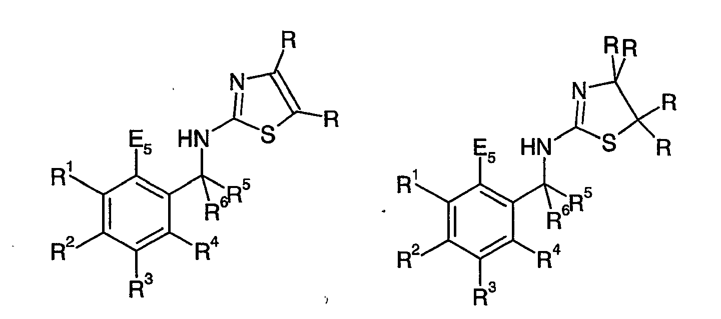 worin R¹, R², R³, R⁴, R⁵ und R⁶ sowie R die vorstehend genannten Bedeutungen aufweisen.

Die Verbindungen der Formeln Ia und Ib können nach beliebigen, dem Fachmann bekannten Verfahren hergestellt werden. In einer bevorzugten Ausführungsform werden die Verbindungen durch Umsetzung einer Verbindung der allgemeinen Formel IIa oder IIb mit einer Verbindung der allgemeinen Formel III hergestellt, wobei Verbindungen der Formel IVa oder IVb gebildet werden. Diese Verbindungen der Formeln IVa und IVb, sowie bevorzugte Verbindungen dieser Formeln sind ebenfalls Gegenstand der vorliegenden Anmeldung.

Um aus den Verbindungen der Formeln IVa bzw. IVb die erfindungsgemäßen Verbindungen der Formeln Ia bzw. Ib zu erhalten, erfolgt eine Reduktion der Verbindungen der Formeln IVa bzw. IVb.

Ein weiterer Gegenstand der vorliegenden Anmeldung ist daher ein Verfahren zur Herstellung von Verbindungen der Formeln Ia und Ib, wobei eine Verbindung der allgemeinen Formel IIa oder IIb mit einer Verbindung der allgemeinen Formel III zu einer Verbindung der allgemeinen Formel IVa oder IVb umgesetzt wird (Schritt a)). Die Verbindung der allgemeinen Formel IVa oder IVb wird anschließend zu einer Verbindung der allgemeinen Formel Ia oder Ib reduziert (Schritt b)).

Die Reaktionsschritte a) und b) sind in dem nachfolgenden Schema dargestellt:
a) 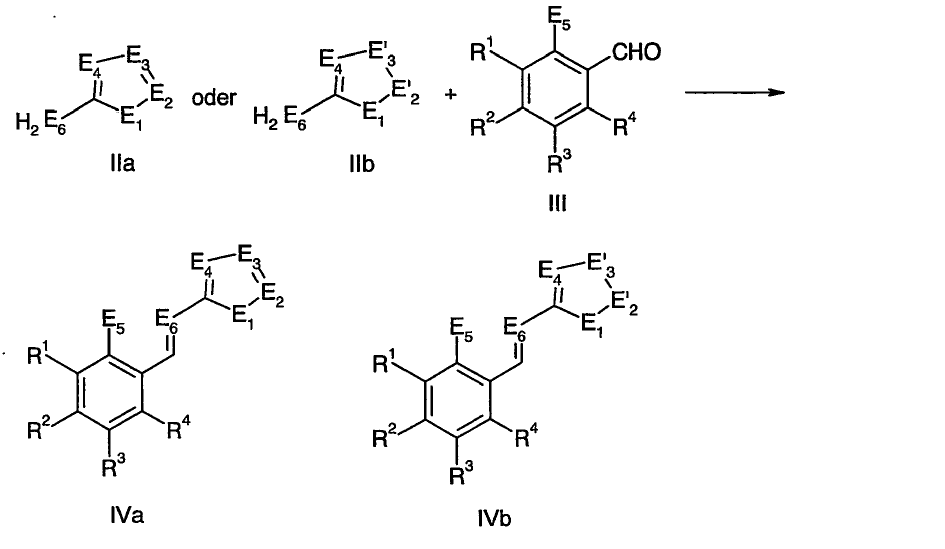
b)

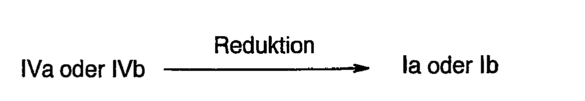

Die Symbole E1, E2, E3, E2', E3', E4, E5, E6, R⁵, R⁶, R¹, R², R³, R⁴, R und R' haben die vorstehend genannten Bedeutungen.

Die Herstellung der Verbindungen der allgemeinen Formeln Ia und Ib erfolgt bevorzugt in zwei Schritten, Schritt a) und Schritt b), wie bereits vorstehend erwähnt.

### Schritt a):

Schritt a) wird im allgemeinen in Lösung durchgeführt. Geeignete Lösungsmittel sind Alkohole wie Methanol, Ethanol und Isopropanol und aromatische Kohlenwasserstoffe wie Toluol, bevorzugt ist Ethanol. Dabei wird üblicherweise die Verbindung der Formel IIa oder IIb in einem der genannten Lösungsmittel vorgelegt und unter Rühren mit dem Aldehyd der allgemeinen Formel III, bevorzugt tropfenweise, versetzt. Der Aldehyd der Formel III wird in einer bevorzugten Ausführungsform ebenfalls in einem der genannten Lösungsmittel, bevorzugt in demselben, in dem die Verbindung der Formel IIa oder IIb vorgelegt wird, gelöst. Im Anschluss an die Zugabe werden katalytische Mengen einer Base oder Säure, bevorzugt Piperidin, Pyridin oder Triethylamin bzw. Ameisensäure, Schwefelsäure oder Toluolsulfonsäure, zugegeben. Anschließend wird die Reaktionslösung auf im allgemeinen 10 bis 150°C, bevorzugt 20 bis 80°C, besonders bevorzugt 40 bis 60°C erhitzt. Das Erhitzen der Reaktionslösung erfolgt dabei im allgemeinen über einen Zeitraum von 0,5 bis 36 Stunden, bevorzugt 1 bis 16 Stunden, besonders bevorzugt 1 bis 4 Stunden. Anschließend wird die Reaktionsmischung auf -60 bis +30°C, bevorzugt 10 bis 20°C abgekühlt. Dabei fällt im allgemeinen ein Feststoff aus, der, bevorzugt durch Filtrieren, abgetrennt wird. Weiteres Produkt wird erhalten, wenn die nach Filtrieren erhaltene Mutterlauge eingeengt und weiter auf im allgemeinen -60 bis +30°C, bevorzugt 10 bis 20°C abgekühlt wird. Anschließend werden die vereinigten Feststoffe, bevorzugt im Vakuum, getrocknet.

In dem anschließenden Schritt b) erfolgt eine Reduktion der erhaltenen Verbindungen der allgemeinen Formeln IVa bzw. IVb.

### Schritt b)

Die Reduktion der Verbindungen der allgemeinen Formel IVa bzw. IVb kann im allgemeinen auf beliebige, dem Fachmann bekannte, Art und Weise erfolgen. Bei der Reduktion sind drei grundsätzlich verschiedene Wege denkbar.
i) Reduktion zu Verbindungen der Formeln Ia bzw. Ib, worin R⁵ und R⁶ Wasserstoff sind,
ii) Reduktion zu Verbindungen der Formeln Ia bzw. Ib, worin R⁵ oder R⁶ Wasserstoff und der jeweils andere Rest ein linearer, verzweigter oder cyclischer Alkylrest oder ein Arylrest ist,
iii) Reduktion zu Verbindungen der Formeln Ia bzw. Ib, worin sowohl R⁵ als auch R⁶ unabhängig voneinander lineare, verzweigte oder cyclische Alkylreste oder Arylreste (unabhängig voneinander) darstellen.

### Weg i):

Die Reduktion erfolgt im allgemeinen mit einem beliebigen, dem Fachmann bekannten Reduktionsmittel. Geeignete Reduktionsmittel sind NaBH₄ und LiAlH₄. Dabei wird die Reduktion im allgemeinen in einem Lösungsmittel, bevorzugt in Methanol, Tetrahydrofuran oder Diethylether durchgeführt. Das molare Verhältnis der Verbindungen der Formel IVa bzw. IVb zu dem eingesetzten Reduktionsmittel beträgt im Allgemeinen 1:1 bis 1:1000, bevorzugt 1:2 bis 1:20. Die Reaktionsdauer beträgt im allgemeinen 0,5 bis 12 Stunden, bevorzugt 1 bis 2 Stunden. Die Aufarbeitung der Reaktionsmischung zur Isolierung des gewünschten Produktes erfolgt nach dem Fachmann bekannten Methoden.

### Weg ii):

Zur Herstellung der Verbindungen der allgemeinen Formeln Ia bzw. Ib gemäß Weg ii) erfolgt eine Umsetzung der Verbindungen der Formeln IVa bzw. IVb mit Metallalkyle. Geeignete Metallalkyle sind abhängig von den gewünschten Resten R⁵ bzw. R⁶. Bevorzugt werden Methyllithium oder Butyllithium eingesetzt. Im allgemeinen erfolgt die Reaktion in einem Lösungsmittel, bevorzugt Tetrahydrofuran der Diethylether. Das molare Verhältnis von Verbindungen der Formel IVa bzw. IVb zu dem Metallalkyl beträgt im Allgemeinen 1:0,5 bis 1:100, bevorzugt 1:1 bis 1:2. Die Reaktion erfolgt im allgemeinen bei Temperaturen von -80 bis + 80°C, bevorzugt -30 bis +20°C. Dabei wird üblicherweise eine Lösung des Metallalkyls zu einer Lösung einer Verbindung der allgemeinen Formel IVa bzw. IVb getropft. Die Reaktionsmischung wird anschließend im allgemeinen langsam auf Raumtemperatur erwärmt und im allgemeinen 1 bis 8 Stunden, bevorzugt 1 bis 2 Stunden nachgeführt. Anschließend erfolgt eine Hydrolyse, bevorzugt unter Eiskühlung mit äquimolaren Mengen eines Alkohols, bevorzugt Methanol. Die gewünschte Verbindung der allgemeinen Formeln Ia bzw. Ib wird anschließend nach dem Fachmann bekannten Methoden isoliert. Das erhaltene Rohprodukt wird anschließend bevorzugt aus einem unpolaren Lösungsmittel, zum Beispiel Pentan, umkristallisiert.

### Weg iii):

Zur Herstellung von Verbindungen der allgemeinen Formel Ia bzw. IIa, in denen sowohl R⁵ als auch R⁶ ein linearer, verzweigter oder cyclischer Alkylrest oder ein Arylrest sind, werden anstelle von Verbindungen der Formeln IVa und IVb Verbindungen der Formeln IV'a und IVb als Ausgangsprodukte eingesetzt, die durch Umsetzung von Verbindungen der Formeln IIa oder IIb mit Ketonen der Formel III' erhalten werden können: 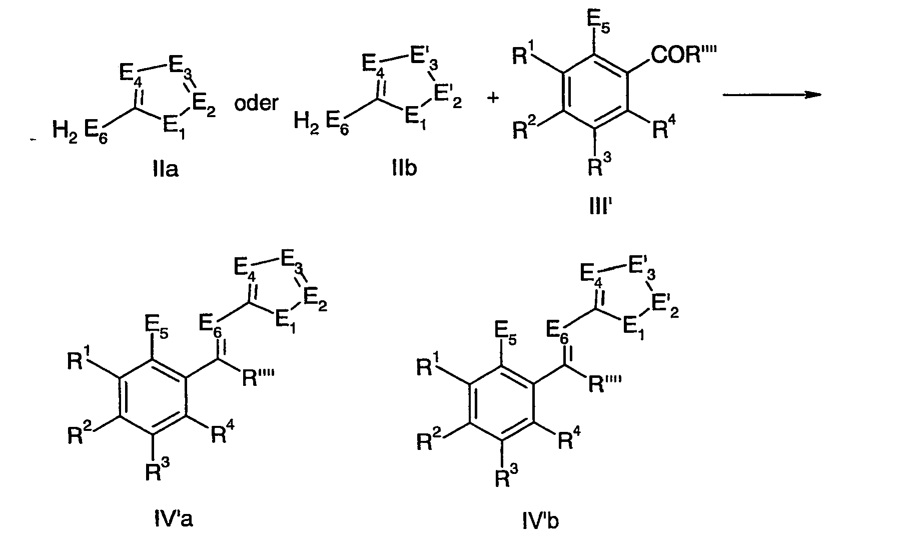 worin R"" ein linearer, verzweigter oder cyclischer Alkylrest oder ein Arylrest, bevorzugt ein linearer C₁- bis C₄-Alkylrest, ist.

Diese Verbindungen der Formeln IV'a und IV'b, die ebenfalls Gegenstand der vorliegenden Anmeldung sind, werden mit weiterem Metallalkyl umgesetzt. Die Umsetzung erfolgt bevorzugt wie in Weg ii) beschrieben.

Die erfindungsgemäßen Liganden eignen sich zur Herstellung von Metallkomplexen. Der Vorteil der erfindungsgemäßen Liganden ist, dass sie über die Gruppe E4 eine zusätzliche Koordinationsstelle im Liganden aufweisen, die in den üblicherweise eingesetzten Liganden nicht vorhanden ist. Diese bewirkt eine zusätzliche Donor-Akzeptor-Wechselwirkung, die das Metallzentrum in einem entsprechenden Metallkomplex stabilisieren kann. Dabei ist es besonders vorteilhaft, dass durch Auswahl der Gruppen E1, E2 bzw. E2', E3 bzw. E3' und E4 (elektronenschiebend oder elekronenziehend) die Nukleophilie des Metallzentrums eingestellt werden kann. Dadurch können die Liganden an jeweilige Metallatome angepasst werden.

Ein weiterer Gegenstand der vorliegenden Anmeldung ist daher die Verwendung der erfindungsgemäßen Verbindung der allgemeinen Formel Ia und Ib zur Herstellung von Metallkomplexen.

Ein weiterer Gegenstand der vorliegenden Erfindung ist ein Metallkomplex der allgemeinen Formel V

LₓMR"_{y}Y_{z} (V)

worin L ein von den Verbindungen gemäß einer der allgemeinen Formeln Ia oder Ib gemäß der vorliegenden Erfindung abgeleiteter mono- oder dianionischer Ligand ist,
worin
- im Falle eines dianionischen Liganden -
- E5: O-, S-, RN-, bevorzugt O-, und
- E6: N-, P-, bevorzugt N-, bedeuten und
- im Falle eines monoanionischen Liganden -
entweder
- E5: O-, S-, RN-, bevorzugt O-, und
- E6: NR, PR, bevorzugt NR, bedeuten oder
- E5: OR, SR, NRR', bevorzugt SR, und
- E6: N-, P-, bevorzugt N-, bedeuten
und die weiteren Symbole E1, E2, E2', E3, E3', E4, R⁵, R⁶, R¹, R², R³, R⁴, R und R' der allgemeinen Formeln Ia und Ib dieselbe Bedeutung aufweisen, die bereits vorstehend angegeben ist;
und
- in dem Fall, dass L ein dianionischer Ligand ist -
- M: Ti, Zr, Hf, V, Nb, Ta, Cr, Mo, W, bevorzugt Ti, Zr, Hf,
- R": Wasserstoff, ein Kohlenwasserstoffrest, bevorzugt ein linearer, verzweigter oder cyclischer Alkylrest, wie bereits vorstehend definiert, NR"'₂, OR"', Halogen, Acetylacetonat, bevorzugt Halogen, OR"', wobei R'" Wasserstoff oder ein linearer oder verzweigter oder cyclischer Alkylrest oder Arylrest ist, wobei geeignete lineare, verzweigte oder cyclische Alkylreste und Arylreste bereits vorstehend sind,
- Y: eine Lewis-Base, ausgewählt aus der Gruppe bestehend aus Tetrahydrofuran, Diethylether, Pyridin und Triethylamin,
- x: 1 oder 2, bevorzugt 1,
- y: 1 bis 4, bevorzugt 2,
- z: 0 bis 2, bevorzugt 0
bedeuten, wobei R" und Y zu einem gemeinsamen Rest, zum Beispiel über eine Alkylengruppe, verbunden sein können und 2x + y die Wertigkeit von M ergibt;
oder
- in dem Fall, dass L ein monoanionischer Ligand ist -
- M: Ti, Zr, Hf, V, Nb, Ta, Cr, Mo, W, Ni, Pd, Co, Fe, Cu, Ru, Rh, bevorzugt Ti, Zr, Hf, Pd, Ni,
- R": Wasserstoff, ein Kohlenwasserstoffrest, bevorzugt ein linearer, verzweigter oder cyclischer Alkylrest, wie vorstehend definiert, NR"'₂, OR"', Halogen, Acetylacetonat, bevorzugt Halogen, OR"', wobei R"' Wasserstoff oder ein linearer, verzweigter oder cyclischer Alkylrest oder Arylrest ist, wobei bevorzugte lineare, verzweigte oder cyclische Alkylreste oder Arylreste vorstehend genannt sind,

- Y: eine Lewis-Base ausgewählt aus der Gruppe bestehend aus Tetrahydrofuran, Diethylether, Pyridin und Triethylamin,
- x: 1, 2 oder 3, bevorzugt 1 oder 2,
- y: 1 bis 4,
- z: 0 bis 2, bevorzugt 0, bedeuten,
wobei R" und Y, zum Beispiel über eine Alkylengruppe, zu einem gemeinsamen Rest verbunden sein können und x + y die Wertigkeit von M ergibt.

Besonders bevorzugt ist der Ligand L in dem erfindungsgemäßen Metallkomplex ein dianionischer Ligand und ganz besonders bevorzugt ist er ein Metallkomplex, worin L ein dianionischer Ligand und M Ti, Zr oder Hf ist, wobei Ti, Zr oder Hf ganz besonders bevorzugt in der Oxidationsstufe IV vorliegen. In diesen ganz besonders bevorzugten Metallkomplexen, worin L ein dianionischer Ligand ist, M Ti, Zr oder Hf ist, wobei M ganz besonders bevorzugt in der Oxidationsstufe IV vorliegt, sind in einer bevorzugten Ausführungsform x 1, y 2 und z 0.

In einer weiteren Ausführungsform des erfindungsgemäßen Verfahrens weist der erfindungsgemäße Metallkomplex mindestens einen monoanionischen Liganden L auf und M ist bevorzugt Ti, Zr, Hf, Ni oder Pd. In dem Fall, wenn M Ti, Zr oder Hf ist, worin diese Metalle M bevorzugt in der Oxidationsstufe IV vorliegen, ist x bevorzugt 2, y bevorzugt 2 und z bevorzugt 0 oder x bevorzugt 1, y bevorzugt 3 und z bevorzugt 0. Wenn M Ni oder Pd ist, und besonders bevorzugt in der Oxidationsstufe 2 vorliegt, ist x bevorzugt 1, y bevorzugt 1 und z bevorzugt 0.

Ganz besonders bevorzugt sind Metallkomplexe der folgenden Formeln:

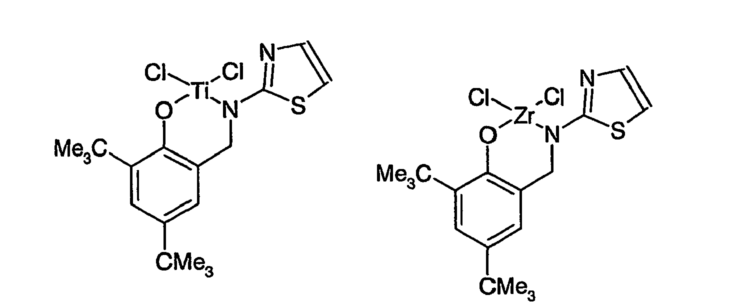

Die erfindungsgemäßen Metallkomplexe werden im allgemeinen durch Deprotonierung einer Verbindung der allgemeinen Formel Ia oder Ib mit einer Base und anschließende Umsetzung mit einer Metallverbindung, oder
durch direkte Umsetzung einer Verbindung der Formel Ia oder Ib mit einer Metallverbindung erhalten,
wobei die Metallverbindung ein Metall M ausgewählt aus der Gruppe bestehend aus Ti, Zr, Hf, V, Nb, Ta, Cr, Mo und W, bevorzugt Ti, Zr, Hf in dem Fall, dass L ein dianionischer Ligand ist, oder ein Metall M ausgewählt aus der Gruppe bestehend aus Ti, Zr, Hf, V, Nb, Ta, Cr, Mo, W, Ni, Pd, Co, Fe, Cu, Ru und Rh, bevorzugt Ti, Zr, Hf, Ni, Pd, in dem Fall, dass L ein monoanionischer Ligand ist, enthält.

Geeignete Metallverbindungen sind Verbindungen der allgemeinen Formel VI

MXₖ (VI)

Darin ist M ein Metall ausgewählt aus der Gruppe bestehend aus Ti, Zr, Hf, V, Nb, Ta, Cr, Mo und W, bevorzugt Ti, Zr und Hf, für den Fall, dass als Ligand L ein dianionischer Ligand eingesetzt wird, bzw. - für den Fall, dass L ein monoanionischer Ligand ist -, ist M ausgewählt aus der Gruppe bestehend aus Ti, Zr, Hf, V, Nb, Ta, Cr, Mo, W, Ni, Pd, Co, Fe, Cu, Ru und Rh, bevorzugt Ti, Zr, Hf, Ni und Pd.
k ist eine Zahl, die die Oxidationsstufe von M sättigen kann, insbesondere eine Zahl von 0 bis 6. So ist k zum Beispiel 2 in dem Fall, dass ein Metall mit der Oxidationsstufe II eingesetzt wird, k ist 3 für den Fall, dass ein Metall mit der Oxidationsstufe III eingesetzt wird, k ist 4 für den Fall, dass ein Metall mit der Oxidationsstufe IV eingesetzt wird usw. Besonders bevorzugt ist k 4 für den Fall, dass Ti(IV), Zr(IV) oder Hf(IV) eingesetzt wird und k ist 3 für den Fall, dass Ti(III) eingesetzt wird. k ist bevorzugt 2 für den Fall, dass Ni(II) oder Pd(II) eingesetzt werden.

X in Formel VI bedeutet Wasserstoff, Halogen, Kohlenwasserstoff, eine Sauerstoff enthaltende Gruppe, eine Schwefel enthaltende Gruppe, eine Stickstoff enthaltende Gruppe, eine Bor enthaltende Gruppe, eine Aluminium enthaltende Gruppe, eine Phosphor enthaltende Gruppe, eine Halogen enthaltende Gruppe, ein heterocyclischer Rest, eine Silikon enthaltende Gruppe, eine Germanium enthaltende Gruppe oder eine Zinn enthaltende Gruppe.

Geeignete Halogenatome sind Fluor, Chlor, Brom und Iod.

Geeignete Kohlenwasserstoffreste sind dieselben Reste, die bereits R¹, R², R³ und R⁴ aufgeführt wurden.

Geeignete heterocyclische Reste sind Stickstoff enthaltende Heterocyclen wie Pyrole, Pyridine, Pyrimidine, Chinoline und Triazine, Sauerstoff enthaltende Verbindungen wie Furan und Pyran, Schwefel enthaltende Verbindungen wie Thiophen, wobei eines oder mehrere Wasserstoffatome in diesen heterocyclischen Verbindungen durch Alkylgruppen oder Alkoxygruppen substituiert sein können.

Geeignete Sauerstoff enthaltende Gruppen sind zum Beispiel Hydroxygruppen, Alkoxygruppen wie Methoxy, Ethoxy, Propoxy und Butoxy, Aryloxygruppen wie Phenoxy, Methylphenoxy, Dimethylphenoxy und Naphthoxy, Arylalkoxygruppen wie Phenylmethoxy und Phenylethoxy, Acetoxygruppen und Carbonylgruppen.

Geeignete Schwefel enthaltende Gruppen sind zum Beispiel Sulfonatogruppen wie Methylsulfonato, Trifluormethansulfonato, Phenylsulfonato, Benzylsulfonato, p-Toluolsulfonato, Trimethylbenzolsulfonato, Trüsobutylbenzolsulfonato, p-Chlorbenzolsulfonato und Pentafluorobenzolsulfonato, Sulfinatogruppen wie Methylsulfinato, Phenylsulfinato, Benzylsulfinato, p-Toluolsulfinato, Trimethylbenzol-sulfinato und Pentafluorobenzolsulfinato, Alkylthiogruppen und Arylthiogruppen.

Geeignete Stickstoff enthaltende Gruppen sind zum Beispiel Aminogruppen, Alkylaminogruppen wie Methylamino, Dimethylamino, Diethylamino, Dipropylamino, Dibutylamino und Dicyclohexylamino und Arylamino oder Alkylarylaminogruppen wie Phenylamino, Diphenylamino, Ditolylamino, Dinaphthylamino und Methylphenylamino.

Geeignete Bor enthaltende Gruppen sind zum Beispiel BR""₄, wobei R"" zum Beispiel Wasserstoff, eine Alkylgruppe, eine Arylgruppe oder ein Halogenatom ist.

Geeignete Phosphor enthaltende Gruppen sind Trialkylphosphingruppen wie Trimethylphosphin, Tributylphosphin und Tricyclohexylphosphin, Triarylphosphingruppen wie Triphenylphosphin und Tritolylphosphin, Phosphitgruppen wie Methylphosphit, Ethylphosphit und Phenylphosphit, Phosphonsäuregruppen und Phosphorsäuregruppen.

Geeignete Silicium enthaltende Gruppen sind zum Beispiel Kohlenwasserstoff substituierte Silylgruppen wie Phenylsilyl, Diphenylsilyl, Trimethylsilyl, Triethylsilyl, Tripropylsilyl, Tricyclohexylsilyl, Triphenylsilyl, Methyldiphenylsilyl, Tritolylsilyl und Trinaphthylsilyl, Kohlenwasserstoff substituierte Silylethergruppen wie Trimethylsilylether, Silicon substituierte Alkylgruppen wie Trimethylsilylmethyl und Silicon substiuierte Arylgruppen wie Trimethylsilylphenyl.

Geeignete Germanium enthaltende Gruppen sind zum Beispiel Gruppen, in denen Silicium in den genannten Silicium enthaltenden Verbindungen durch Germanium ersetzt ist.

Geeignete Zinn enthaltende Verbindungen sind zum Beispiel Verbindungen, in denen Silicium in den genannten Silicium enthaltenden Verbindungen durch Zinn ersetzt ist.

Geeignete Halogen enthaltende Gruppen sind Fluor enthaltende Gruppen wie PF₆, Chlor enthaltende Gruppen wie ClO₄ und SbCl₆ und Iod enthaltende Gruppen wie IO₄.

Geeignete Aluminium enthaltende Verbindungen sind AIR""'₄, worin R""' zum Beispiel Wasserstoff, ein Alkylgruppe oder eine Arylgruppe ist, die gegebenenfalls substituiert ist.

Besonders bevorzugte Gruppen X sind Halogenatome wie Chlor und Brom, bevorzugt Chlor, Alkylgruppen wie Methyl, Alkoxygruppen wie Propoxy (insbesondere i-Propoxy), Alkylaminogruppen wie Dimethylamino und Kohlenwasserstoffgruppen wie Cyclopentadienylreste, die gegebenenfalls mit einer oder meheren Alkylgruppen, insbesondere Methyl oder tert-Butyl substituiert sind.

Es ist möglich, dass die Metallverbindung mehrere verschiedene der genannten Gruppen X enthält. Besonders bevorzugte Metallverbindungen sind Ti(NMe₂)₄, (tert-BuCp)TiCl₃, Ti(NMe₂)₂Cl₂, Ti(Oi-Pr)₄ sowie die entsprechenden Hf- und Zr-Verbindungen (Cp bedeutet Cyclopentadienyl). Des weiteren sind Verbindungen wie TiCl₃, TiCl₄, Ti(CH₂C₆H₅)₄, Ti(NMe₃)₄ sowie die entsprechenden Zr- und Hf-Verbindungen geeignet. Weiterhin sind NiCl₂, NiBr₂, PdCl₂ sowie PdBr₂ geeignet. Es können auch Komplexe der genannten Metallverbindungen mit THF (Tetrahydrofuran), Acetonitril oder Diethylether eingesetzt werden.

Das molare Verhältnis zwischen der erfindungsgemäß eingesetzten Verbindung der Formel Ia oder Ib und der Metallverbindung der Formel VI beträgt im allgemeinen 0,5 bis 2 zu 1, bevorzugt 1 bis 1,2 zu 1.

### Herstellung der erfindungsgemäßen Metallkomplexe gemäß Formel V durch Deprotonierung einer Verbindung der Formel Ia oder Ib und anschließende Umsetzung mit einer Metallverbindung:

Die zur Deprotonierung der Verbindung der allgemeinen Formel Ia oder Ib eingesetzte Base ist ausgewählt aus der Gruppe bestehend aus Metallalkylen, zum Beispiel n-Butyllithium, und Metallhydriden, z.B. Natriumhydrid. Die Menge der eingesetzten Base ist abhängig davon, ob ein monoanionischer oder dianionischer Ligand aus den Verbindungen der Formel Ia oder Ib hergestellt wird. Im Falle der Herstellung eines monoanionischen Liganden beträgt das Verhältnis der Verbindung der Formel Ia oder Ib zur eingesetzten Base (molares Verhältnis) 1 zu 0,5 bis 1,5, bevorzugt 1 zu 1. Im Falle der Herstellung eines dianionischen Liganden beträgt das Verhältnis der Verbindung der Formel Ia oder Ib zur eingesetzten Base (molares Verhältnis) im allgemeinen 1 zu 1 bis 3, bevorzugt 1 zu 1 bis 2.

Die Deprotonierung erfolgt nach dem Fachmann bekannten Verfahren in einer Inertgasatmosphäre. Die Deprotonierung erfolgt im allgemeinen in einem Lösungsmittel augewählt aus der Gruppe bestehend aus Ether, Tetrahydrofuran und Toluol. Die benötigten Lösungsmittel werden im allgemeinen vor der Benutzung absolutiert. Üblicherweise wird zu einer auf im allgemeinen -60 bis +10 °C, bevorzugt -20 °C gekühlten Lösung der Verbindung der Formel Ia oder Ib die Base tropfenweise zugegeben. Anschließend wird langsam auf Raumtemperatur erwärmt. Die nachfolgende Aufarbeitung erfolgt nach dem Fachmann bekannten Verfahren.

Das erhaltene Metallsalz, bevorzugt ein Lithiumsalz, wird mit einer der vorstehend genannten Metallverbindungen der Formel VI umgesetzt. Das Verhältnis von Metallsalz zur Metallverbindung beträgt im allgemeinen 1 zu 1 bis 1 zu 4, bevorzugt 1 zu 1 bis 1 zu 2. Die Umsetzung erfolgt nach dem Fachmann bekannten Verfahren, im allgemeinen durch Vorlegen des Metallsalzes und der eingesetzten Metallverbindung und anschließende Zugabe eines unpolaren Lösungsmittels, zum Beispiel Pentan, unter Kühlung auf im allgemeinen -60 bis +20°C, bevorzugt -20 bis 0°C. Nach anschließendem Erwärmen der Reaktionsmischung auf Raumtemperatur und Nachrühren wird gebildetes, ausgefallenes Metallsalz im allgemeinen abfiltriert und der gewünschte Metallkomplex isoliert.

### Direkte Umsetzung einer Verbindung der Formel Ia oder Ib mit einer Metallverbindung:

Bei der direkten Umsetzung wird im allgemeinen die Metallverbindung der Formel VI in einem Lösungsmittel, zum Beispiel Toluol, vorgelegt und bei einer Temperatur von im allgemeinen -60 bis + 60°C, bevorzugt -20 bis + 20°C mit einer Verbindung der Formel Ia oder Ib, ebenfalls in einem Lösungsmittel gelöst, tropfenweise versetzt. Anschließend wird über einen Zeitraum von im allgemeinen 1 bis 16 Stunden, bevorzugt 1 bis 4 Stunden weitergerührt. Die Aufarbeitung erfolgt nach dem Fachmann bekannten Verfahren. Die gesamte Reaktion wird in einer Inertgasatmosphäre durchgeführt.

Die erfindungsgemäßen Metallkomplexe, die sich aufgrund der erfindungsgemäßen Liganden insbesondere dadurch auszeichnen, dass sie eine hohe Stabilität besitzen, eignen sich hervorragend als Polymerisationskatalysatoren, insbesondere zur Polymerisation von Olefinen. Somit ist Gegenstand der vorliegenden Erfindung ebenfalls die Verwendung der erfindungsgemäßen Metallkomplexe in der Polymerisation von Olefinen.

Damit die erfindungsgemäßen Metallkomplexe eine genügend hohe katalytische Aktivität in der Polymerisation von Olefinen aufweisen, werden die Metallkomplexe üblicherweise gemeinsam mit einem Cokatalysator eingesetzt, wobei "in-situ" die katalytisch aktive Spezies aus dem Metallkomplex gebildet wird.

Ein weiterer Gegenstand der vorliegenden Anmeldung ist daher eine katalytisch aktive Zusammensetzung umfassend
a) einen erfindungsgemäßen Metallkomplex der allgemeinen Formel V, als Komponente A,
b) mindestens eine Verbindung, als Komponente B, ausgewählt aus der Gruppe bestehend aus
   b1) einer organometallischen Verbindung, als Komponente B1,
   b2) einer Organoaluminium-oxy-Verbindung, als Komponente B2, und
   b3) einer Verbindung, die mit dem Metallkomplex unter Ausbildung eines Ionenpaares reagiert, als Komponente B3.

### Komponente B

### Komponente B1: Organometallische Verbindung

Geeignete organometallische Verbindungen (B1), die in der katalytisch aktiven Zusammensetzung gemäß der vorliegenden Erfindung eingesetzt werden können, umfassen organometallische Verbindungen, die mindestens ein Metall der Gruppen I, II, XII und XIII des Periodensystems der Elemente enthalten.

Beispielsweise sind Organoaluminiumverbindungen der folgenden allgemeinen Formel geeignet:

R^{a}ₘAl(OR^{b})ₙHₚZ_{q}

worin R^{a} und R^{b} unabhängig voneinander ein Kohlenwasserstoffrest mit 1 bis 15 Kohlenstoffatomen, bevorzugt mit 1 bis 4 Kohlenstoffatomen ist, Z ein Halogenatom ist und m, n, p und q den folgenden Bedingungen genügen: 0 < m ≤ 3, 0 ≤ n < 3, 0 ≤ p < 3, 0 ≤ q < 3 und m + n + p + q =3.

Weiterhin geeignet sind Alkylkomplexverbindungen, die ein Metall der Gruppe I und Aluminium enthalten, entsprechend der nachfolgenden Formel:

M²AlR^{a}₄

worin M² Li, Na oder K ist, und R^{a} ein Kohlenwasserstoffrest mit 1 bis 15 Kohlenstoffatomen, bevorzugt 1 bis 4 Kohlenstoffatomen darstellt.

Weiterhin sind die Alkylverbindungen von Metallen der Gruppen II oder XII des Periodensystems der Elemente geeignet, gemäß der folgenden Formel

R^{a} R^{b}M³

worin R^{a} und R^{b} unabhängig voneinander ein Kohlenwasserstoffrest mit 1 bis 15 Kohlenwasserstoffatomen, bevorzugt 1 bis 4 Kohlenstoffatomen sind, und M³ Mg Zn oder Cd ist.

Beispiele für geeignete Organoaluminiumverbindungen sind Organoaluminiumverbindungen ausgewählt aus der Gruppe bestehend aus Tri-n-alkylaluminium wie Trimethylaluminium, Triethylaluminium, Tri-n-butylaluminium, Tripropylaluminium, Tripentylaluminium, Trihexylaluminium, Trioctylaluminium und Tridecylaluminium, verzweigte Trialkylaluminiumverbindungen wie Triisopropylaluminium, Triisobutylaluminium, Tri-sec-butylaluminium, Tri-tert-butylaluminium, Tri-2-methyl-butylaluminium, Tri-3-methylbutylaluminium, Tri-2-methylpentylaluminium, Tri-3-methylpentylaluminium, Tri-4-methylpentylaluminium, Tri-2-methylhexylaluminium, Tri-3-methylhexylaluminium und Tri-2-ethylhexylaluminium, Tricycloalkylaluminiumverbindungen wie Tricyclohexylaluminium und Tricyclooctylaluminium, Triarylaluminiumverbindungen wie Triphenylaluminium und Tritolylaluminium, Dialkylaluminiumhydride wie Diethylaluminiumhydrid und Düsobutylaluminiumhydrid, Trialkenylaluminiumverbindungen, zum Beispiel solche, die durch die Formel (i-C₄H₉)ₓAl_{y}(C₅H₁₀)₂, worin x, y und z jeweils eine positive Zahl darstellen und z ≥ 2x ist, wie Isoprenylaluminium, Alkylaluminiumalkoxide wie Isobutylaluminiummethoxit, Isobutylaluminiumethoxid und Isobutylaluminiumisopropoxid, Dialkylaluminiumalkoxide wie Dimethylaluminiummetoxid, Dimethylaluminiumetoxid und Dibutylaluminiumbutoxid, Alkylaluminiumsesquialkoxide wie Ethylaluminiumsesquiethoxid, Butylaluminiumsequibutoxid sowie teilweise alkoxylierte Alkylaluminiumverbindungen mit einer mittleren Zusammensetzung der Formel R^{a}_{2,5}Al(OR^{b})_{0,5}, worin R^{a} und R^{b} die vorstehend genannte Bedeutung haben, Dialkylaluminiumaryloxide wie Diethylaluminiumphenoxid, Diethylaluminium(2,6-di-t-butyl-4-methylphenoxid), Ethylaluminium- bis(2,6-Di-t-butyl-4-methylphenoxid), Diisobutylaluminium(2,6-di-t-butyl-4-methylphenoxid) und Isobutylaluminium-bis(2,6-di-t-butyl-4-methylphenoxid), Dialkylaluminiumhalide wie Dimethylaluminiumchlorid, Diethylaluminiumchlorid, Dibutylaluminiumchlorid, Diethylaluminiumbromid und Düsobutylaluminiumchlorid, Alkylaluminiumsesquihalide wie Ethylaluminiumsesquichlorid, Butylaluminiumsesquichlorid und Ethylaluminiumsesquibromid, partiell halogenierte Alkylaluminiumverbindungen wie Ethylaluminiumdichlorid, Propylaluminiumdichlorid und Butylaluminiumdibromid, Dialkylaluminiumhydride wie Diethylaluminiumhydrid und Dibutylaluminiumhydrid, partiell hydrierte Alkylaluminiumverbindungen, zum Beispiel Alkylaluminiumdihydride wie Ethylaluminiumdihydrid und Propylaluminiumdihydrid und partiell alkoxylierte und halogenierte Alkylaluminiumverbindungen wie Ethylaluminiumethoxychlorid, Butylaluminiumbutoxychlorid und Ethylaluminiumethoxybromid.

Weiterhin geeignete Organoaluminiumverbindungen sind solche, worin zwei oder mehr Aluminiumverbindungen zum Beispiel über ein Stickstoffatom kombiniert sind, wie (C₂H₅)₂AlN(C₂H₅)Al(C₂H₅)₂.

Geeignete Alkylkomplexverbindungen enthaltend ein Metall der Gruppe I und Aluminium sind LiM(C₂H₅)₄ und LW(e₇H₁₅)₄.

Weitere geeignete organometallische Verbindungen (B1) umfassen Methyllithium, Ethyllithium, Propyllithium, Butyllithium, Methylmagnesiumbromid, Methylmagnesiumchlorid, Ethylmagnesiumbromid, Ethylmagnesiumchlorid, Propylmagnesiumbromid, Propylmagnesiumchlorid, Butylmagnesiumbromid, Butylmagnesiumchlorid, Dimethylmagnesium, Diethylmagnesium, Dibutylmagnesium und Butylethylmagnesium.

Des weiteren sind Verbindungen geeignet, die die oben genannten Organoaluminiumverbindungen während der Polymerisation bilden, zum Beispiel eine Kombination von halogenierten Aluminiumverbindungen und Alkylmagnesium.

Besonders bevorzugt werden als organometallische Verbindungen (B1) Organoaluminiumverbindungen eingesetzt.

Die organometallischen Verbindungen (B1) können einzeln oder in Kombination von zwei oder mehreren der Verbindungen eingesetzt werden.

### B2: Organoaluminium-oxy-Verbindung

Die Organoaluminium-oxy-Verbindungen (B2), die in den erfindungsgemäßen katalytisch aktiven Zusammensetzungen eingesetzt werden können, können übliche Aluminoxane oder benzolunlösliche Organoaluminium-oxy-Verbindungen sein, wie sie zum Beispiel in JP-A 78687/1990 offenbart sind.

Übliche Aluminoxane können zum Beispiel durch die folgenden Verfahren hergestellt werden und werden im allgemeinen als Lösung in einem Kohlenwasserstoff erhalten.
(i) Zugabe einer Organoaluminiumverbindung wie Trialkylaluminium zu einer Suspension einer Verbindung, die Adsorbtionswasser enthält, oder einem Salz, das Kristallisationswasser enthält, zum Beispiel Magnesiumchloridhydrat, Kupfersulfathydrat, Aluminiumsulfathydrat, Nickelsulfathydrat oder Cerchloridhydrat in einem Kohlenwasserstoff, so dass die Organoaluminiumverbindung mit dem Adsorbtionswasser oder Kristallisationswasser reagieren kann.
(ii) Umsetzung einer Organoaluminiumverbindung wie Trialkylaluminium mit Wasser, Eis oder Wasserdampf in einem Lösungsmittel wie Benzol, Toluol, Ethylether oder Tetrahydrofuran.
(iii) Reaktion eines Organozinnoxids wie Dimethylzinnoxid oder Dibutylzinnoxid mit einer Organoaluminiumverbindung wie Trialkylaluminium in einem Lösungsmittel wie Decan, Benzol oder Toluol.

Das Aluminoxan kann geringe Mengen einer organometallischen Verbindung enthalten. Des weiteren ist es möglich, dass das Lösungsmittel oder die nicht abreagierte Organoaluminiumverbindung von der erhaltenen Aluminoxanlösung abdestilliert wird und der Rückstand in einem Lösungsmittel wieder gelöst wird oder in einem für Aluminoxane schlechten Lösungsmittel suspendiert wird.

Geeignete Organoaluminiumverbindungen, die zur Herstellung der Aluminoxane eingesetzt werden, sind dieselben, die bereits vorstehend als Organoaluminiumverbindungen (B1) beschrieben sind. Bevorzugt sind Trialkylaluminiumverbindungen und Tricycloalkylaluminiumverbindungen. Besonders bevorzugt ist Trimethylaluminium.

Die Organoaluminiumverbindungen können einzeln oder in Kombination von zwei oder mehreren verschiedenen Verbindungen genutzt werden.

Geeignete Lösungsmittel zur Herstellung der Aluminoxane umfassen aromatische Kohlenwasserstoffe wie Benzol, Toluol, Xylol, Cumol und Cymol, aliphatische Kohlenwasserstoffe wie Pentan, Hexan, Heptan, Octan, Decan, Dodecan, Hexadecan und Octadecan, alicyclische Kohlenwasserstoffe wie Cyclopentan, Cyclohexan, Cyclooctan und Methylcyclopentan, Petroleumfraktionen wie Gasolin, Kerosin und Gasöl, und Halogenverbindungen dieser aromatischen, aliphatischen und alicyclischen Kohlenwasserstoffe, insbesondere Chloride und Bromide davon. Des weiteren sind Ether wie Ethylether und Tetrahydrofuran als Lösungsmittel geeignet. Besonders bevorzugt werden aromatische Kohlenwasserstoffe und aliphatische Kohlenwasserstoffe eingesetzt.

Die benzol-unlösliche Organoaluminium-oxy-Verbindung ist bevorzugt eine Organoaluminium-oxy-Verbindung enthaltend eine Al-Komponente, die bei 60°C in Benzol in einer Menge von nicht mehr als 10%, bevorzugt nicht mehr als 5%, besonders bevorzugt nicht mehr als 2%, löslich ist, bezogen auf das Al-Atom. Das heißt, die benzolunslösliche Organoaluminium-oxy-Verbindung ist bevorzugt unlöslich oder nahezu unlöslich in Benzol.

Des weiteren können beispielsweise Organoaluminium-oxy-Verbindungen eingsetzt werden, die Bor enthalten, wie sie in EP-A 0 950 667 offenbart sind.

Die genannten Organoaluminium-oxy-Verbindungen (B2) können einzeln oder in Kombination von zwei oder mehreren dieser Verbindungen eingesetzt werden.

### B3: Verbindung, die mit dem Metallkomplex unter Ausbildung eines Ionenpaares reagiert

Geeignete Verbindungen sind alle Verbindungen, die im Kontakt mit dem Übergangsmetallkomplex (A) ein Ionenpaar bilden. Geeignete Verbindungen sind Lewis-Säuren, ionische Verbindungen, Boranverbindungen und Carboranverbindungen, wie in JP-A 501950/1989, JP-A 502036/1989, JP-A 179005/1991, JP-A 179006/1991, JP-A 207703/1991 und JP-A 207704/1991 und US 5,321,106 offenbart. Des weiteren können Heteropolyverbindungen und Isopolyverbindungen eingesetzt werden.

Geeignete Lewis-Säuren sind zum Beispiel Verbindungen der Formel BR₃, worin R Fluor oder eine Phenylgruppe ist, die mit Fluor, Methyl oder Trifluoromethyl substituiert sein kann. Geeignete Verbindungen sind Trifluorobor, Triphenylbor, Tris(4-fluorophenyl)bor, Tris(3,5-difluorophenyl)bor, Tris(4-fluoromethylphenyl)bor, Tris(pentafluorophenyl)bor, Tris(p-tolyl)bor, Tris(o-tolyl)bor und Tris(3,5-dimethylphenyl)bor.

Geeignete ionische Verbindungen sind Verbindungen der allgemeinen Formel VII 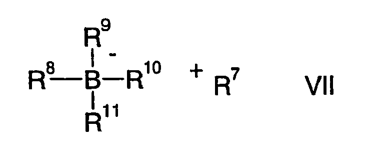 worin R⁷H⁺, ein Carboniumkation, ein Oxoniumkation, Ammoniumkation, ein Phosphoniumkation, ein Cycloheptyltrienylkation oder ein Ferroceniumkation, enthaltend ein Übergangsmetall, ist. R⁸ bis R¹¹ sind unabhängig voneinander eine organische Gruppe, bevorzugt eine Arylgruppe oder eine substituierte Arylgruppe.

R⁷ ist besonders bevorzugt ein Carboniumkation oder ein Ammoniumkation, ganz besonders bevorzugt Triphenylcarbonium, N,N-Dimethylanilinium oder N,N-Diethylanilium. Des weiteren sind als ionische Verbindungen trialkylsubstituierte Ammoniumsalze, N,N-Dialkylaniliniumsalze, Dialkylammoniumsalze und Triarylphosphoniumsalze geeignet.

Geeignete trialkylsubstituierte Ammoniumsalze sind Triethylammoniumtetra(phenyl)bor, Tripropylammoniumtetra(phenyl)bor, Tri(n-butyl)ammoniumtetra(phenyl)bor, Tri(n-butyl)ammoniumtetra(pentafluorophenyl)bor, Tripropylammoniumtetra(o, p-dimethylphenyl)bor, Tri(n-butyl)ammoniumtetra(p-trifluoromethylphenyl)bor, Tri(n-butyl)-ammoniumtetra(3,5-ditrifluoromethylphenyl)bor und Tri(n-butyl)ammoniumtetra(o-tolyl)-bor.

Geeignete N,N-Dialkylaniliniumsalze umfassen N,N-Dimethylaniliniumtetra(phenyl)bor, N,N-Diethylaniliniumtetra(phenyl)bor und N,N-2,4,6-Pentamethylaniliniumtetra(phenyl)-bor.

Geeignete Dialkylammoniumsalze sind Di(1-propyl)ammoniumtetra-(pentafluorophenyl)bor und Dicycloammoniumtetra(phenyl)bor.

Weitere geeignete ionische Verbindungen sind Triphenylcarbeniumtetrakis-(pentafluorophenyl)borat, N,N-Dimethylaniliniumtetrakis(pentaffuorophenyl)borat, und Ferroceniumtetra(pentafluorophenyl)borat.

Weitere geeignete Verbindungen, die mit dem Metallkomplex unter Ausbildung eines Ionenpaares reagieren, sind in EP-A 0 950 667 offenbart. Ganz besonders bevorzugt wird Triphenylcarboniumtetrakis(pentafluorophenyl)borat eingesetzt.

Die katalytisch aktive Zusammensetzung gemäß der vorliegenden Anmeldung umfassend die Komponenten A und B kann zusätzlich ein Trägermaterial als Komponente C enthalten. Solche geträgerten katalytisch aktiven Zusammensetzungen eignen sich insbesondere zum Einsatz in Gasphasenpolymerisationsverfahren, ganz besonders bevorzugt in Gasphasenwirbelschichtpolymerisationsverfahren.

### Trägermaterial, C

Als Trägermaterial sind sowohl anorganische als auch organische Verbindungen geeignet.

Bevorzugte anorganische Verbindungen sind poröse Oxide, anorganische Chloride, Ton, Tonmineralien und Schichtverbindungen.

Geeignete poröse Oxide umfassen SiO₂ Al₂O₃, MgO, ZrO, TiO₂, B₂O₃ CaO, ZnO, BaO, ThO₂ und Mischungen von Verbindungen, die diese Oxide enthalten, wie natürliche oder synthetische Zeolithe, SiO₂-MgO SiO₂-Al₂O₃, SiO₂-TiO₂ SiO₂-V₂O₅ SiO₂-Cr₂O₃ und SiO₂-TiO₂-MgO Besonders bevorzugt sind poröse Oxide, die SiO₂ und/oder Al₂O₃ als Hauptkomponenten enthalten.

Die anorganischen Oxide können des weiteren geringe Mengen Carbonate, Sulfate, Nitrate oder Oxide enthalten.

Bevorzugt haben die porösen Oxide, die gemäß der vorliegenden Erfindung eingesetzt werden, einen Teilchendurchmesser von 10 bis 300 µm, besonders bevorzugt 20 bis 200 µm und eine spezifische Oberfläche von im allgemeinen 50 bis 1000 m²/g, bevorzugt 100 bis 700 m²/g und eine Porenvolumen von im allgemeinen 0,3 bis 3 cm³/g. Das Trägermaterial kann vor Einsatz bei im allgemeinen 100 bis 1.000°C, bevorzugt 150 bis 700°C calciniert werden, falls notwendig.

Geeignete anorganische Oxide, Ton, Tonmineralien und Schichtverbindungen sind zum Beispiel in EP-A 0 950 667 offenbart.

Geeignete organische Trägermaterialien sind zum Beispiel körnige oder partikelförmige feste Verbindungen mit einem Partikeldurchmesser von im allgemeinen 10 bis 300 µm. Beispiele für solche Verbindungen umfassen (Co)polymere, die durch Umsetzung eines α-Olefins mit 2 bis 14 Kohlenstoffatomen wie Ethylen, Propylen, 1-Buten oder 4-Methyl-1-Penten als Hauptmonomeren hergestellt wurden, (Co)polymere, die durch Reaktion von Vinylcyclohexan oder Styrol als Hauptmonomere hergestellt wurden und Derivate der genannten (Co)polymere.

Die erfindungsgemäßen katalytisch aktiven Zusammensetzungen sind hervorragend zur Polymerisation und Copolymerisation von Olefinen geeignet. Aufgrund der vielseitig variierbaren und einfach herstellbaren Liganden sind eine Vielzahl verschiedener Metallkomplexe und somit eine Vielzahl verschiedener katalytisch aktiver Zusammensetzungen erhältlich, die zur Herstellung maßgeschneiderter Polymere oder Copolymere eingesetzt werden können.

Ein weiterer Gegenstand der vorliegenden Anmeldung ist daher die Verwendung einer erfindungsgemäßen katalytisch aktiven Zusammensetzung zur Polymerisation oder Copolymerisation von Olefinen.

Ein weiterer Gegenstand der vorliegenden Erfindung ist ein Verfahren zur Polymerisation oder Copolymerisation von Olefinen, wobei ein Olefin in Gegenwart einer erfindungsgemäßen katalytisch aktiven Zusammensetzung polymerisiert wird oder mindestens zwei verschiedene Olefine in Gegenwart einer erfindungsgemäßen katalytisch aktiven Zusammensetzung copolymerisiert werden.

Die Durchführung der Polymerisation oder Copolymerisation sowie geeignete Vorrichtungen zur Durchführung der Polymerisation oder Copolymerisation und geeignete Olefine sind dem Fachmann bekannt.

Bevorzugt eingesetzte geeignete Olefine sind ausgewählt aus der Gruppe bestehend aus α-Olefinen mit 2 bis 20 Kohlenstoffatomen wie Ethylen, Propylen, 1-Buten, 1-Penten, 3-Methyl-1-buten, 1-Hexen, 4-Methyl-1-penten, 3-Methyl-1-penten, 1-Octen, 1-Decen, 1-Dodecen, 1-Tetradecen, 1-Hexadecen und 1-Octadecen, Cycloolefine mit 3 bis 20 Kohlenstoffatomen wie Cyclopenten, Cyclohepten, Norbonen, 5-Methyl-2-norbonen und Tetracyclododecen, polare Monomere wie α, β-ungesättige Carbonsäuren wie Acrylsäure, Methacrylsäure, Fumarsäure, Maleinsäureanhydrid, Itaconsäure, Itaconsäureanhydrid und Bicyclo[2.2.1]-5-hepten-2,3-dicarbonsäure, Metallsalze dieser Säuren wie Natriumsalze, Kaliumsalze, Lithiumsalze, Zinksalze, Magnesiumsalze und Calciumsalze, α, βungesättigte Carbonsäureester wie Methylacrylat, Ethylacrylat, n-Propylacrylat, Isopropylacrylat, n-Butylacrylat, Isobutylacrylat, tert-Butylacrylat, 2-Ethylhexylacrylat, Methylmethacrylat, Ethylmethacrylat, n-Propylmethacrylat, Isopropylmethacrylat, n-Butylmethacrylat und Isobutylmethacrylat, Vinylester, Vinylacetat, Vinylpropionat, Vinylcaproat, Vinylcaprat, Vinyllaurat, Vinylstearath und Vinyltrifluoroacetat, ungesättigte Glycidylester und halogenierte Olefine wie Vinylchlorid, Vinylfluorid und Allylfluorid.

Vinylcyclohexane, Diene und Polyene sind ebenfalls als Olefine einsetzbar. Geeignete Diene und Polyene sind cyclisch oder linear und weisen 4 bis 30 Kohlenstoffatome, bevorzugt 4 bis 20 Kohlenstoffatome auf und haben zwei oder mehr Doppelbindungen. Geeignete Verbindungen sind zum Beispiel Butadien, Isopren, 4-Methyl-1,3-pentadien, 1,3-Pentadien, 1,4-Pentadien, 1,5-Hexadien, 1,4-Hexadien und 1,3-Hexadien.

Aromatische Vinylverbindungen sind ebenfalls als Olefine einsetzbar. Geeignete aromatische Vinylverbindungen sind zum Beispiel Styrol, Mono- oder Polyalkylstyrole wie o-Methylstyrol, m-Methylstyrol, p-Methylstyrol, o, p-Dimethylstyrol, o-Ethylstyrol, m-Ethylstyrol und p-Ethylstyrol, funktionelle Gruppen enthaltende Styrolderivate wie Methoxystyrol, Ethoxystyrol, Hydroxystyrol, o-Chlorstyrol, p-Chlorstyrol und Divinylbenzol sowie weitere Verbindungen wie 3-Phenylpropylen, 4-Phenylpropylen und β-Methylstyrol.

Weitere geeignete α-Olefine sind zum Beispiel in EP-A 0 950 667 offenbart.

Bevorzugt werden als α-Olefine Ethylen und Propylen, besonders bevorzugt Ethylen eingesetzt. Die α-Olefine können allein oder in Kombination zweier oder mehrerer verschiedener α-Olefine eingesetzt werden. Weitere bevorzugt eingesetzte Olefine sind Styrol, iso-Buten, interne Olefine wie 2-Buten, cyclische Olefine wie Norbonen oder Cyclopenten und polare Olefine wie Acrylate.

Des weiteren ist es möglich, ein α-Olefin und ein polares Olefin, zum Beispiel solche, die vorstehend beschrieben wurden, cozupolymerisieren.

Des weiteren ist es möglich, ein α-Olefin gemäß der vorliegenden Anmeldung und ein nicht konjugiertes Dien oder Polyen cozupolymerisieren. Beispiele für nicht-konjugierte Diene und Polyene umfassen 1,4-Pentadien, 1,5-Hexadien und 1,4-Hexadien.

Es ist ebenfalls möglich, weitere der genannten Monomere in einer Copolymerisation mit α-Olefinen einzusetzen.

Die Polymerisation oder Copolymerisation kann nach beliebigen, dem Fachmann bekannten Verfahren durchgeführt werden. Geeignete Zugabereihenfolgen und Verfahren sind zum Beispiel in EP-A 0 950 667 genannt. So ist es möglich, die Komponente (A) (Metallkomplex) und mindestens eine Komponente (B) ausgewählt aus einer organometallischen Verbindung (B1), einer Organoaluminium-oxy-Verbindung (B2) und einer Verbindung, die mit dem Metallkomplex unter Ausbildung eines Ionenpaares reagiert (B3) in einer beliebigen Reihenfolge dem Polymerisationsreaktor zuzuführen. Es ist ebenfalls möglich, zunächst einen Katalysator durch in Kontakt bringen der Komponenten (A) und (B) herzustellen und diesen anschließend in den Polymerisationsreaktor einzubringen. Des weiteren ist es möglich, einen durch in Kontakt bringen der Komponenten (A) und (B) hergestellten Katalysator gemeinsam mit weiterer Komponente (B), die von der ersten Komponente (B) unterschiedlich sein kann, in beliebiger Reihenfolge in den Polymerisationsreaktor einzuführen. Des weiteren ist es möglich, den Metallkomplex (A) aufgebracht auf einem Trägermaterial (C), und die Komponente (B) in beliebiger Reihenfolge in den Polymerisationsreaktor einzuführen. Es ist ebenfalls möglich, dass die Komponenten (A) und (B) gemeinsam auf einem Trägermaterial (C) aufgebracht sind, und in dieser Form in den Polymerisationsreaktor eingeführt werden.

Des weiteren sind zahlreiche weitere Varianten denkbar, beispielsweise die in EP-A 0 950 667 offenbarten Varianten.

Des weiteren ist es möglich, die erfindungsgemäßen katalytisch aktiven Zusammensetzungen nicht allein sondern auch in Verbindung mit anderen katalytisch aktiven Zusammensetzungen des gleichen Typs oder anderen Polymerisationskatalysatoren, zum Beispiel Philipps-Katalysatoren, Ziegler-Katalysatoren und/oder Metallocen-Katalysatoren einzusetzen, die jeweils ebenfalls in geträgerter oder ungeträgerter Form vorliegen können.

In einer weiteren Ausführungsform der vorliegenden Erfindung kann das Olefin auf den festen Katalysatorkomponenten vorpolymerisiert werden, wobei der Metallkomplex (A) und gegebenenfalls Komponente (B) auf einem Träger (C) aufgebracht sind.

Die Polymerisation kann als Lösungspolymerisation, Suspensionspolymerisation oder als Gasphasenpolymerisation durchgeführt werden.

Geeignete Lösungsmittel für die Lösungspolymerisation sind Kohlenwasserstoffe, zum Beispiel aliphatische Kohlenwasserstoffe wie Propan, Butan, Pentan, Hexan, Heptan, Octan, Decan, Dodecan und Kerosin, alicyclische Kohlenwasserstoffe wie Cyclopentan, Cyclohexan und Methylcyclopentan, aromatische Kohlenwasserstoffe wie Benzol, Toluol und Xylol, halogenierte Kohlenwasserstoffe wie Ethylenchlorid, Chlorbenzol und Dichlormethan und Mischungen der genannten Kohlenwasserstoffe. Es ist ebenfalls möglich, dass das Olefin selbst als Lösungsmittel eingesetzt wird.

Der Metallkomplex (A) wird im allgemeinen in einer Menge von 10⁻¹² bis 10⁻² mol, bevorzugt 10⁻¹⁰ bis 10⁻³ mol, bezogen auf ein Liter Reaktionsvolumen eingesetzt. Gemäß der vorliegenden Erfindung kann das Olefin mit hoher Polymerisätionaktivität polymerisiert werden, auch wenn der Metallkomplex (A) in relativ geringen Konzentrationen eingesetzt wird.

Die Komponente (B1) wird im allgemeinen in einer solchen Menge eingesetzt, dass das Molverhältnis der Komponente (B1) zu dem Übergangsmetallatom (M) in dem Metallkomplex (A) im allgemeinen 0,01 bis 100000, bevorzugt 0,05 bis 50000 beträgt.

Die Komponente (B2) kann in einer solchen Menge eingesetzt werden, dass das molare Verhältnis des Aluminiumatoms in Komponente (B2) zu dem Übergangsmetallatom (M) in dem Metallkomplex (A) im allgemeinen 10 bis 500000, bevorzugt 20 bis 100000 beträgt.

Die Komponente (B3) kann in einer solchen Menge eingesetzt werden, dass das molare Verhältnis der Komponente (B3) zu dem Übergangsmetallatom (M) in dem Metallkomplex (A) im allgemeinen 1 bis 10, bevorzugt 1 bis 5 beträgt.

Die Polymerisationstemperatur in dem erfindungsgemäßen Verfahren beträgt im allgemeinen -50 bis 200°C, bevorzugt 0 bis 170°C. Der Polymerisationsdruck beträgt im allgemeinen Atmosphärendruck bis 100 bar, bevorzugt Atmosphärendruck bis 50 bar. Die Polymerisation kann im Batch-Verfahren, halb kontinuierlich oder kontinuierlich durchgeführt werden. Es ist ebenfalls möglich, die Polymerisation in zwei oder mehreren getrennten Schritten unter unterschiedlichen Reaktionsbedingungen durchzuführen.

Das Molekulargewicht der erhaltenen Olefin(co)polymere kann durch die Anwesenheit von Wasserstoff im Polymerisationssystem oder durch Wechsel der Polymerisationstemperatur reguliert werden. Des weiteren ist es möglich, das Molekulargewicht durch Wechsel der Art der Komponente (B) zu regulieren.

Somit sind mit dem erfindungsgemäßen Verfahren zahlreiche Polymere und Copolymere zugänglich, deren Eigenschaften je nach Art der eingesetzten katalytisch aktiven Zusammensetzungen und der Reaktionsbedingungen unterschiedlich sind. Ein weiterer Gegenstand der vorliegenden Erfindung sind somit Polymere oder Copolymere herstellbar nach dem erfindungsgemäßen Verfahren.

Die nachfolgenden Beispiele erläutern die Erfindung zusätzlich.

### Beispiele

### 1. Allgemeines

Aufgrund der Oxidations- und Hydrolyseempfindlichkeit der Metallkomplexe erfolgten alle Darstellungsexperimente der Metallverbindungen sowie die Polymerisationsversuche unter einer Inertgasatmosphäre unter Benutzung der Schlenk-Technik. Die hierfür benötigten Lösungsmittel wurden vor der Benutzung absolutiert. Die Ausgangsverbindungen für die Ligandenherstellung sowie NaBH₄, Ti(Oi-Pr)₄, die BuLi-, MeLi- und MgCl(CH₂Ph)-Lösungen wurden von Acros bzw. Aldrich bezogen und ohne zusätzliche Reinigungsschritte verwendet. Die weiteren verwendeten Metallprecursor wurden nach den in der Literatur beschriebenen Methoden hergestellt. Zur Aktivierung der Komplexe für die Polymerisationsversuche wurde eine MAO-Lösung in Toluol mit einem Aluminiumgehalt von 10% bzw. 7% verwendet.

### 2. Synthese der Liganden

### 2.1 Darstellung der Thiazolyliminomethylphenole

Zur Lösung eines Aminothiazols in Ethanol wurde unter Rühren bei Raumtemperatur eine Hydroxybenzaldehyd-Lösung in Ethanol zugetropft. Nach beendeter Zugabe wurden einige Tropfen Piperidin zugegeben, und die Reaktionslösung für 2 h unter Rückfluss erhitzt. Anschließend wurde auf 0°C abgekühlt. Der dabei ausgefallene gelbe Feststoff wurde abfiltriert. Durch Einengen der Mutterlauge und Kühlen auf -30°C wurde weiteres Produkt ausgefällt. Die vereinigten Fraktionen wurden im Vakuum getrocknet. **2.1.1 4,6-Di-*tert*-butyl-2-(thiazol-2'-yliminomethyl)-phenol (1)** 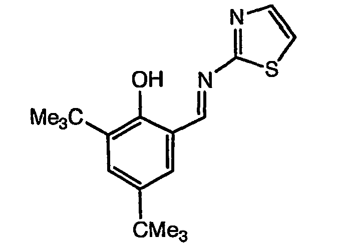
- **Ansatz:**: 2.71 g (27.1 mmol) 2-Aminothiazol in 30 mL EtOH 6.35 g (27.1 mmol) 3,5-Di-tert-butyl-2-hydroxybenzaldehyd in 40 mL EtOH 80 mg Piperidin
- **Ausbeute:**: 68% (5.87 g, 18.5 mmol)
**¹H-NMR** (400 MHz, C₆D₆): δ = 1.21 (s, 9 H, C4-C(CH₃)₃), 1.59 (s, 9 H, C6-C(CH₃)₃), 6.43 (d, ³*J*_{HH} = 3.6 Hz, 1 H, NCH=CHS), 6.94 (d, ⁴*J*_{HH} = 2.4 Hz, 1 H, C5-H), 7.36 (d, ³*J*_{HH} = 3.6 Hz, 1 H, NC*H*=CHS), 7.58 (d,⁴*J*_{HH} = 2.4 Hz, 1 H, C3-H), 9.14 (s, 1 H, CH=N), 13.29 (s,1 H, OH).

### 2.1.2 4,6-Di-tert-butyl-2-(4'-methylthiazol-2'-yliminomethyl)-phenol (2)

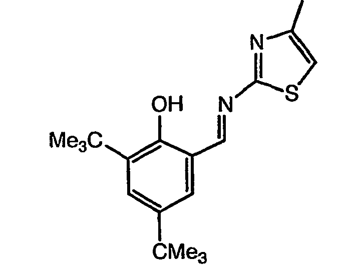

- **Ansatz:**: 3.90 g (34.1 mmol) 2-Amino-4-methylthiazol in 30 mL EtOH 8.00 g (34.1 mmol) 3,5-Di-tert-butyl-2-hydroxybenzaldehyd in 40 mL EtOH 100 mg Piperidin
- **Ausbeute:**: 76% (8.56 g, 25.9 mmol)
**¹H-NMR** (400 MHz, C₆D₆): δ = 1.22 (s, 9 H, C4-C(CH₃)₃), 1.59 (s, 9 H, C6-C(CH₃)₃), 2.20 (s, 3 H, CH₃), 6.14 (s, 1 H, NC(CH₃)=C*H*S), 6.95 (d, ⁴*J*_{HH} = 2.4 Hz, 1 H, C5-H), 7.59 (d, ⁴*J*_{HH} = 2.4 Hz,1 H, C3-H), 9.17 (s, 1H, CH=N), 13.36 (s, 1 H, OH).

### 2.1.3 4,6-Di-tert-butyl-2-(5'-methylthiazol-2'-yliminomethyl)-phenol (3)

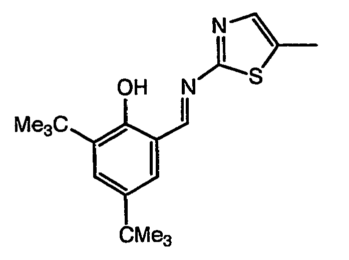

- **Ansatz:**: 3.90 g (34.1 mmol) 2-Amino-5-methylthiazol in 30 mL EtOH 8.00 g (34.1 mmol) 3,5-Di-tert-butyl-2-hydroxybenzaldehyd in 40 mL EtOH 100 mg Piperidin
- **Ausbeute:**: 80% (9.00 g, 27.2 mmol)
**¹H-NMR** (400 MHz, C₆D₆): δ = 1.21 (s, 9 H, C4-C(CH₃)₃), 1.59 (s, 9 H, C6-C(CH₃)₃), 1.82 (s, 3 H, CH₃), 6.96 (d, ⁴*J*_{HH} = 2.4 Hz, 1 H, C3-H), 7.10 (s, 1H, NC*H*=C(CH₃)S), 7.59 (d, ⁴*J*_{HH} = 2.4 Hz,1H, C5-H), 9.18 (s,1H, CH=N), 13.36 (s, 1 H, OH).

### 2.1.4 4,6-Di-tert butyl-2-(benzothiazol-2'-yliminomethyl)-phenol (4)

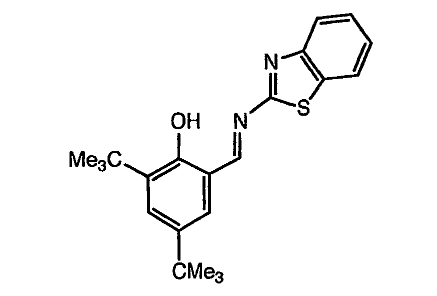

- **Ansatz:**: 5.13 g (34.1 mmol) 2-Aminobenzothiazol in 30 mL EtOH 8.00 g (34.1 mmol) 3,5-Di-tert-butyl-2-hydroxybenzaldehyd in 40 mL EtOH 100 mg Piperidin
- **Ausbeute:**: 71% (8.87 g, 24.2 mmol)
**¹H-NMR** (400 MHz, C₆D₆): δ = 1.22 (s, 9 H, C4-C(CH₃)₃), 1.58 (s, 9 H, C6-C(CH₃)₃), 6.94 (d, ⁴*J*_{HH} = 2.0 Hz, 1 H, C3-H), 6.97 (t, ³*J*_{HH} = 7.6 Hz, 1 H, benzothiazolyl-C6-H), 7.18 (1H, benzotiazolyl-C5-H), 7.32 (d, ³*J*_{HH} = 7.6 Hz , 1 H, benzothiazolyl-C7-H), 7.61 (d, ⁴*J*_{HH} = 2.0 Hz, 1H, C5-H), 8.00 (d, ³*J*_{HH} = 8.0 Hz, 1 H, bezothiazolyl-C4-H), 9.15 (s, 1 H, CH=N), 13.29 (s, 1H, OH).

### 2.2 Reduktion der Thiazolylimintimethylphenole

Der Lösung eines Thiazolyliminomethylphenols in Methanol wurde unter Rühren bei Raumtemperatur portionenweise NaBH₄ zugegeben. Nach beendeter Zugabe wurde 1 h weiter gerührt. Währenddessen entfärbte sich die Reaktionsmischung und ein farbloser Feststoff fiel aus. Das Produkt wurde abfiltriert und im Vakuum getrocknet.

### 2.2.1 [Lig1]H₂ (5) (Reduktion von Verbindung (1) aus Beispiel 2.1.1)

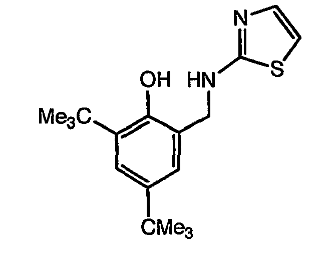

- **Ansatz:**: 1.47 g (4.64 mmol) **1** in 10 mL MeOH 292 mg (7.72 mmol) NaBH₄
- **Ausbeute:**: 85% (1.26 g, 3.96 mmol)
**¹H-NMR** (400 MHz, C₆D₆): δ = 1.40 (s, 9 H, C4-C(CH₃)₃), 1.68 (s, 9 H, C6-C(CH₃)₃), 4.03 (s, 3 H, C*H*₂N*H*), 5.73 (d,³*J*_{HH} = 3.6 Hz, 1H, NCH=CHS), 6.72 (d, *³J*_{HH} = 3.6 Hz, 1 H, NC*H*=CHS), 6.91 (d, ⁴*J*_{HH} = 2.4 Hz, 1H, C3-H), 7.52 (d, ⁴*J*_{HH} = 2.4 Hz, 1H, C5-H), 11.2 (s,1 H, OH).

### 2.2.2 [Lig2]H₂ (6) (Reduktion von Verbindung (2) aus Beispiel 2.1.2)

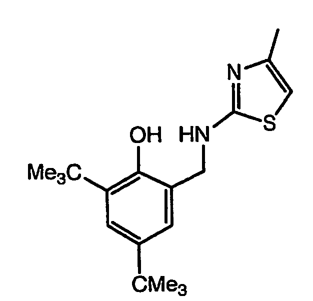

- **Ansatz:**: 8.31 g (25.1 mmol) **2** in 80 mL MeOH 1.6 g (42.3 mmol) NaBH₄
- **Ausbeute:**: 81% (6.79 g, 20.4 mmol)
**¹H-NMR** (400 MHz, C₆D₆): δ = 1.40 (s, 9 H, C4-C(CH₃)₃), 1.68 (s, 9 H, C6-C(CH₃)₃), 2.07 (s, 3 H, CH₃), 4.01 (d, *³J_{HH}* = 6.0 Hz, 2 H, CH₂), 4.07 (t, ³*J*_{HH} = 6.0 Hz, 1 H, NH), 5.41 (s, 1 H, NC(CH₃)=CHS), 6.93 (d, ⁴*J*_{HH} = 2.6 Hz, H, C3-H), 7.52 (d, ⁴*J*_{HH} = 2.6 Hz, 1 H, C5-H),11.42 (s,1 H, OH).

### 2.2.3 [Lig3]H₂ (7) (Reduktion von Verbindung (3) aus Beispiel 2.1.3)

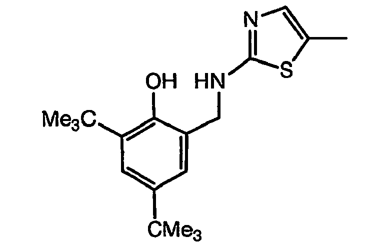

- **Ansatz:**: 8.89 g (26.9 mmol) **3** in 80 mL MeOH 1.63 g (43.1 mmol) NaBH₄
- **Ausbeute:**: 82% (7.30 g, 21.9 mmol)
**¹H-NMR** (400 MHz, C₆D₆): δ = 1.41 (s, 9 H, C4-C(CH₃)₃), 1.70 (s, 3 H, CH₃), 1.71 (s, 9 H, C6-C(CH₃)₃), 3.95 (t, 1 H, NH), 4.04 (d, ³*J*_{HH} = 6.4 Hz, 2 H, CH₂), 6.42 (s, 1 H, NC*H* =C(CH₃)S), 6.93 (d, 1 H, ⁴*J*_{HH} = 2.4 Hz, C3-H), 7.53 (d, ⁴*J*_{HH} = 2.4 Hz, 1 H, C5-H), 11.31 (s,1 H, OH).

### 2.2.4 [Lig4]H₂ (8) (Reduktion von Verbindung (4) aus Beispiel 2.1.4)

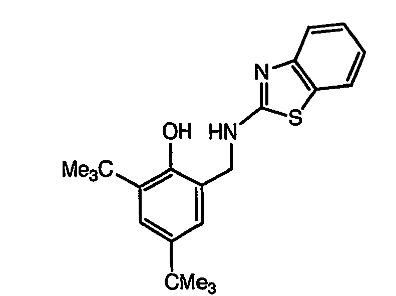

- **Ansatz:**: 5.85 g (15.9 mmol) 4 in 40 mL MeOH 0.97 g (25.6 mmol) NaBH₄
- **Ausbeute:**: 68% (3.98 g, 10.8 mmol)
**¹H-NMR** (400 MHz, C₆D₆): δ = 1.42 (s, 9 H, C4-C(CH₃)₃), 1.69 (s, 9 H, C6-C(CH₃)₃), 4.06 (m, 3 H, CH₂, NH), 6.81 (t, ³*J*_{HH} = 7.6 Hz, 1 H, benzothiazolyl-C6-H), 6.93 (d, ⁴*J*/_{HH} = 2.4 Hz, C3-H), 7.04 (t, ³*J*_{HH} = 7.6 Hz, 1 H, benzotiazolyl-C5-H), 7.10 (d, ³*J*_{HH} = 7.6 Hz, 1 H, benzotiazolyl-C7-H), 7.52 (d, ⁴*J*_{HH} = 2.4 Hz, 1 H, C5-H), 7.63 (d, ³*J*_{HH} = 7.6 Hz, 1 H, bezothiazolyl-C4-H), 11.31 (s, 1 H, OH).

### 2.3 Reaktionen der Thiazolyliminomethylphenole mit Metallalkylen

Zur Lösung des Imins wurde unter Rühren bei -70°C die Lösung des Metallalkyls zugetropft. Die Reaktionsmischung wurde langsam auf Raumtemperatur erwärmt und weitere 15 h gerührt. Unter Eiskühlung wurde dann vorsichtig mit äquimolarer Menge Methanol hydrolysiert. Anschließend wurde verdünnte wässerige NH₄Cl-Lösung zugegeben. Die Phasen wurden getrennt. Nach dem Trocknen der etherischen Phase mit MgSO₄ wurde das Lösungsmittel unter reduziertem Druck entfernt. Das Rohprodukt wurde aus Pentan umkristallisiert.

### 2.3.1 [Lig5]H₂ (9) (Umsetzung von Verbindung (1) aus Beispiel 2.1.1)

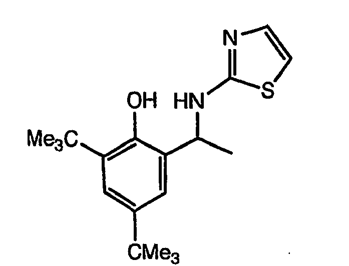

- **Ansatz:**: 0.50 g (1.58 mmol) **1** in 35 mL Et₂O 2.0 mL (3.20 mmol) 1.6 M MeLi-Lösung in Et₂O
- **Ausbeute:**: 92% (422 mg, 1.46 mmol)
**¹H-NMIR** (400 MHz, C₆D₆): δ = 1.01 (d, ³*J*_{HH} = 7.2 Hz, 3 H, CH₃), 1.38 (s, 9 H, C4-C(CH₃)₃), 1.71 (s, 9 H, C6-C(CH₃)₃), 4.07 (d, 1 H, NH), 5.23 (quin, 1 H, C*H*(NH)(CH₃)), 5.73 (d, ³*J*_{HH} = 3.6 Hz, 1H, NCH=C*H*S), 6.73 (d, ³*J*_{HH} = 3.6 Hz, 1H, NC*H*=CHS), 7.09 (d, ⁴*J*_{HH} = 2.4 Hz, 1 H, C3-H), 7.49 (d, ⁴*J*_{HH} = 2.4 Hz, 1 H, C5-H), 10.94 (s, 1 H, OH).

### 2.3.2 [Lig6]H₂ (10) (Umsetzung von Verbindung (1) aus Beispiel 2.1.1)

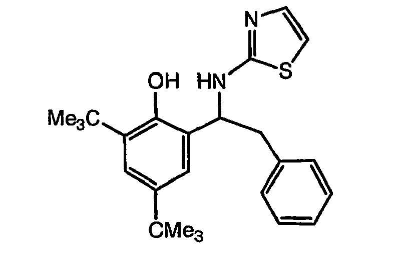

- **Ansatz:**: 1.00 g (3.16 mmol) **1** in 35 mL Et₂O 7.0 mL (7.0 mmol) 1 M MgCl(CH₂Ph)-Lösung in Et₂O
- **Ausbeute:**: 85% (1.10 g, 2.69 mmol)
**¹H-NMR** (400 MHz, C₆D₆): δ = 1.42 (s, 9 H, C4-C(CH₃)₃), 1.69 (s, 9 H, C6-C(CH₃)₃), 2.83 (d, ³*J*_{HH} = 7.6 Hz, 2 H, CH₂Ph), 4.38 (d, ³*J*_{HH} = 6.4 Hz, 1H, NH), 5.45 (quart, ³*J*_{HH} 7.2 Hz, 1 H, C*H*(NH)(CH₂Ph)), 5.65 (d, *³J_{HH}* = 3.6 Hz, 1 H, NCH=C*H*S), 6.67 (d, ³*J*_{HH} = 3.6 Hz, 1 H, NC*H*=CHS), 6.87 (d, ³*J*_{HH} = 7.2 Hz, 2 H, ortho CH₂C₆H₅), 6.92 (t, ³*J*_{HH} = 7.2 Hz, 1 H, para CH₂C₆H₅), 6.70 (t, ³*J*_{HH} = 7.2 Hz, 2 H, meta CH₂C₆H₅), 7.17 (d, ⁴*J_{HH}* = 2.0 Hz, 1 H, C3-H), 7.51 (d, ⁴*J*_{HH} = 2.0 Hz,1H, C5-H),10.87 (s, 1 H, OH).

### 2.3.3 [Lig6]H₂ (11) (Umsetzung von Verbindung (4) aus Beispiel 2.1.4)

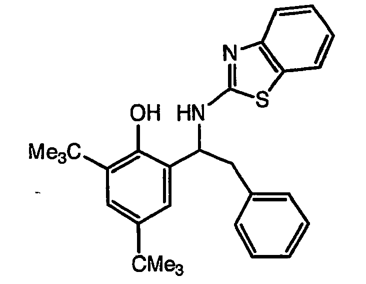

**¹H-NMR** (400 Mm, C₆D₆): δ = 1.45 (s, 9 H, 4-C(CH₃)₃), 1.70 (s, 9 H, 6-C(CH₃)₃), 2.83 (d, ³*J*_{HH} = 7.6 Hz, 2 H, CH₂), 4.45 (d, ³*J*_{HH} = 7.2 Hz, 1 H, NH), 5.63 (quart, ³*J*_{HH} = 7.6 Hz, 1 H, CH), 6.77 (t, ³*J*_{HH} = 7.6 Hz, 1 H, benzothiazolyl-C6-H), 6.95 (m, 7 H, bezothiazolyl-C5-H und -C7-H, CH₂C₆*H*₅), 7.22 (d, ⁴*J*_{HH} = 2.4 Hz, 1 H, C3-H), 7.52 (d, ⁴*J*_{HH} = 2.4 Hz, 1 H, C5-H), 7.58 (d, *³J_{HH}* = 8.0 Hz, 1H, bezothiazolyl-C4-H), 11.13 (s, 1 H, OH).

### 3. Komplexsynthesen

### 3.1 [Lig1]Ti(NMe₂)₂ (12) (Umsetzung von Verbindung (5) aus Beispiel 2.2.1)

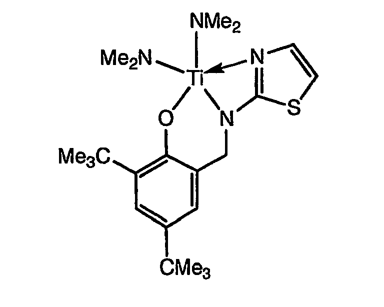

Zur gelben Lösung des Ti(NMe₂)₄ (60 mg, 268 µmol) in 5 mL Toluol wurde unter Rühren bei Raumtemperatur die Lösung des Liganden [Lig1]H₂ (**5**) (86 mg, 270 µmol) in 5 mL Toluol zugetropft. Währenddessen wechselte die Farbe der Reaktionslösung zu dunkelrot. Nach beendeter Zugabe wurde die Lösung 1 h weitergerührt. Anschließend wurden die flüchtigen Bestandteile der Reaktionsmischung unter reduziertem Druck entfernt, der Rückstand wurde mit Pentan aufgenommen und 15 min gerührt. Nach Entfernen des Lösungsmittels wurde das Produkt im Vakuum getrocknet. Man erhielt in quantitativer Ausbeute [Lig1]Ti(NMe₂)₂ (**12**) in Form eines rotbraunen Feststoffes.
**¹H-NMR** (400 MHz, C₆D₆): δ = 1.39 (s, 9 H, C4-C(CH₃)₃), 1.72 (s, 9 H, C6-C(CH₃), 3.15 (s, 12 H, N(CH₃)₂), 4.35 (s, 2 H, CH₂), 5.79 (d, ³*J*_{HH} = 4.0 Hz, 1 H, NCH=CHS), 6.95 (d, ³*J*_{HH} = 4.0 Hz, 1 H, NC*H*=CHS), 7.04 (d, *⁴J*_{HH} = 2.4 Hz, 1 H, C3-H), 7.53 (d, ⁴*J*_{HH} = 2.4 Hz, H, C5-H).

### 3.2 [Lig2]TiCl(tert-BuCp) (14) (Umsetzung von Verbindung (6) aus Beispiel 2.2.2) 3.2.1 (Lig2]Li₂(Et₂O)₂ (13)

Unter Rühren bei -70 °C wurden 0.56 mL (1.38 mmol) der 2.5 M BuLi-Lösung in Ether der Lösung des Liganden [Lig2]H₂ (**6**) (230 mg, 692 µmol) zugetropft. Nach beendeter Zugabe ließ man langsam auf Raumtemperatur erwärmen. Die flüchtigen Bestandteile der Reaktionslösung wurden dann unter reduziertem Druck entfernt und der Rückstand mit Pentan aufgenommen. Durch Abkühlen auf -70 °C erhielt man [LÄg2]Li₂(Et₂O)₂ (13) in Form farbloser Kristalle. Ausbeute: 81% (275 mg, 558 µmol).
**¹H-NMR** (400 MHz, THF-d₈): δ = 1.10 (t, Et₂O), 1.21 (s, 9 H, C4-C(CH₃)₃), 1.30 (s, 9 H, C6-C(CH₃)₃), 1.81 (s, 3 H, CH₃), 3.38 (quart, Et₂O) 4.50 (br, 2 H, CH₂), 5.44 (s, 1 H, NC(CH₃)=C*H*S), 6.83 (d, ⁴*J*_{HH} = 2.4 Hz, H, C3-H), 6.99 (d, *⁴J_{HH}* = 2.4 Hz,1H, C5-H).

### 3.2.2 [Lig2]TiCl(tert-BuCp) (14)

Das Lithium-Salz (**13**) (89 mg, 181 µol) sowie das (*tert*-BuCp)TiCl₃ (50 mg, 181 µmol) wurden vorgelegt. Unter Eiskühlung und Rühren wurden 10 mL vorgekühlten Pentans zugegeben. Die Reaktionsmischung wurde anschließend 2 h bei Raumtemperatur gerührt. Man filtrierte vom ausgefallenen LiCl ab und entfernte dann das Lösungsmittel unter reduziertem Druck. Man erhielt 94 mg (176 µmol, 97 %) des Produktes in Form eines dunkelroten Feststoffes.
**¹H-NMR** (400 MHz, C₆D₆): δ = 1.11 (s, 9 H, CpC(CH₃)₃), 1.31 (s, 9 H, C4-C(CH₃)₃), 1.64 (s, 9 H, C6-C(CH₃)₃), 2.10 (s, 3 H, CH₃), 4.10 (d, ²*J*_{HH} = 16.0 Hz, 1 H, CH₂), 4.60 (d, ²*J*_{HH} = 16.0 Hz, 1 H, CH₂), 5.43 (s, 1 H, NC(CH₃)=CHS), 6.48 (m, 2 H, Cp-H), 6.52 (m, 1H, Cp-H), 6.63 (m, 1 H, Cp-H), 6.91 (d, ⁴*J*_{HH} = 2.2 Hz, 1 H, C3-H), 7.44 (d, ⁴*J*_{HH} = 2.2 Hz, 1 H, C5-H).

### 3.3 {[Ligl]H}₂TiCl₂ (15) (Umsetzung von Verbindung (5) aus Beispiel 2.2.1)

Zur orangen Lösung des Ti(NMe₂)₂Cl₂ (100 mg, 483 µmol) in 8 mL Et₂O wurde unter Rühren bei -70 °C die Lösung des Liganden [Lig1]H₂ (5) (154 mg, 483 µmol) in 8 mL Et₂O zugetropft, wobei die Farbe der Reaktionslösung zu dunkelrot wechselte. Man ließ langsam auf Raumtemperatur erwärmen, währenddessen die flüchtigen Bestandteile der Reaktionsmischung unter reduziertem Druck entfernt wurden. Der dunkelrote Feststoff wurde mit 5 mL kaltem Pentan gewaschen und anschließend im Vakuum getrocknet. Ausbeute (aus NMR): 217 mg (228 µmol, 94%).
**¹H-NMR** (400 MHz, C₆D₆): δ = 1.27 (s, 9 H, C4-C(CH₃)₃), 1.34 (s, 9 H, C6-C(CH₃)₃), 1.68 (s, 9 H, C4-C(CH₃)₃), 1.77 (s, 9 H, C6-C(CH₃)₃), 4.27 (d, ²*J*_{HH} (Spinsystem 1) = 14.8 Hz, 1 H; d, ²*J*_{HH} (Spinsystem 2) = 14.4 Hz, 1 H, CH₂), 4.90 (d, ²*J*_{HH} (Spinsystem 2) = 14.4 Hz, 1 H, CH₂), 5.59 (d, ³*J*_{HH} (Spinsystem 3) = 4.4 Hz, 1 H, NCH=C*H*S), 5.82 (d, ³*J*_{HH} (Spinsystem 4) = 2.8 Hz, 1H, NCH=C*H*S), 5.90 (d, ²*J*_{HH} (Spinsystem 1) = 14.8 Hz, 1 H, CH₂), 6.80 (d, ³*J*_{HH} (Spinsystem 3) = 4.4 Hz, 1 H, NC*H*=CHS), 6.84 (d, ³*J*_{HH} (Spinsystem 4) = 2.8 Hz, H, NC*H*=CHS), 7.25, 7.38, 7.46 und 7.54 (4 H, C3-H, C5-H).

### 3.4 [Lig2]TiCl₂ (16) (Umsetzung von Verbindung (6) aus Beispiel 2.2.2)

Zur Lösung des Ti(NMe₂)₂Cl₂ (75 mg, 362 µmol) in 10 mL THF wurde unter Rühren bei Raumtemperatur die Lösung des Liganden [Lig1]H₂ (**6**) (120 mg, 361 µmol) in 10 mL THF zugetropft. Nach beendeter Zugabe wurde weitere 30 min gerührt, wonach die flüchtigen Bestandteile der Reaktionsmischung unter reduziertem Druck entfernt wurden. Der dunkelrote Feststoff wurde mit 5 mL kaltem Pentan gewaschen und anschließend im Vakuum getrocknet. Ausbeute (aus NMR): 217 mg (228 µmol, 94%).
**¹H-NMR** (400 MHz, THF-*d₈*): δ = 1.31 (s, 9 H, C4-C(CH₃)₃), 1.50 (s, 9 H, C6-C(CH₃)₃), 2.43 (s, 3 H, CH₃), 2.46 (br, HN(C*H*₃)₂), 4.20 (br, *H*N(CH₃)₂), 4.65 (s, 2 H, CH₂), 6.38 (s, 1 H, NC(CH₃)=C*H*S), 7.23 (s, H, C3-H), 7.26 (s,1H, C5-H).

### 3.5 [Lig3]Ti(Oi-Pr)₂ (17) (Umsetzung von Verbindung (7) aus Beispiel 2.2.3)

Zur Lösung von 49 mg (169 µmol) Ti(Oi-Pr)₄ in 4 mL Et₂O wurde unter Rühren bei Raumtemperatur die Lösung des Liganden [Lig3]H₂ (**7**) (56 mg, 169 µmol) in 4 mL Et₂O zugetropft. Nach beendeter Zugabe wurde weitere 2 h gerührt. Anschließend wurden die flüchtigen Bestandteile der Reaktionsmischung unter reduziertem Druck entfernt. Der Rückstand wurde mit Pentan aufgenommen und auf -70 °C gekühlt. Nach ca. 20 h erhielt man 55 mg (111 µmol, 66%) des Komplexes (**17**) in Form eines orangen Feststoffes.
**¹H-NMR** (400 MHz, C₆D₆): δ = 0.82 (d, ³*J*_{HH} = 6.0 Hz, 3 H, OCH(C*H*₃)₂), 1.16 (d, ³*J*_{HH} = 6.0 Hz, 3 H, OCH(C*H*₃)₂), 1.20 (d, *³J_{HH}* = 6.0 Hz, 3 H, OCH(C*H*₃)₂), 1.36 (s, 9 H, C4-C(CH₃)₃), 1.70 (s, 9 H, C6-C(CH₃)₃), 1.81 (s, 6 H, CH₃), 4.38 (d, ²*J*_{HH} = 14.6 Hz, 1 H, CH₂), 4.80 (m,1 H, OC*H*(CH₃)₂), 5.07 (m, H, OC*H*(CH₃)₂), 5.32 (d, ²*J*_{HH} =14.6 Hz, 1 H, CH₂), 7.35 (s, 2 H, NC*H*=CHS, C3-H), 7.46 (d, ⁴*J*_{HH} = 2.4 Hz,1H, C5-H).

### 3.6 {[Lig3]H}₂Ti(Oi-Pr)₂ (18) (Umsetzung von Verbindung (7) aus Beispiel 2.2.3)

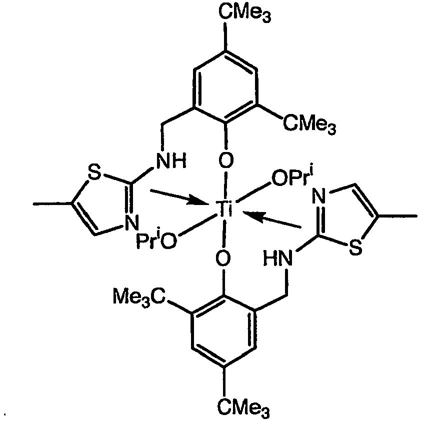

Zur Lösung von 62 mg (218 µmol) Ti(Oi-Pr)₄ in 4 mL Et₂O wurde unter Rühren bei Raumtemperatur die Lösung des Liganden [Lig3]H₂ (**7**) (145 mg, 436 µmol) in 4 mL Et₂O zugetropft. Nach beendeter Zugabe wurde weitere 2 h gerührt. Anschließend wurden die flüchtigen Bestandteile der Reaktionsmischung unter reduziertem Druck entfernt. Man erhielt in quantitativer Ausbeute den Komplexes (**18**) in Form eines gelben Feststoffes.
**¹H-NMR** (400 MHz, C₆D₆): δ = 0.95 (d, ³*J*_{HH} = 6.0 Hz, 12 H, OCH(CH₃)₂), 1.41 (s, 18 H, C4-C(CH₃)₃), 1.54 (s, 6 H, CH₃), 1.80 (s, 18 H, C6-C(CH₃)₃), 3.69 (d, ²*J*_{HH} = 13.2 Hz, 2 H, CH₂), 4.70 (sept, ³*J*_{HH} = 6.0 Hz, 2 H, OC*H*(CH₃)₂), 4.96 (d, ²*J*_{HH} = 13.2 Hz, 2 H, CH₂), 5.65 (s, 2 H, NC*H*=CHS), 7.24 (d, ⁴*J*_{HH} = 2.4 Hz, 2 H, C3-H), 7.55 (d, ⁴*J*_{HH} = 2.4 Hz, 2 H, C5-H),11.77 (s, 2 H, NH).

### 3.7 [LigS]Ti(Oi-Pr)₂ (19) (Umsetzung von Verbindung (9) aus Beispiel 2.3.1)

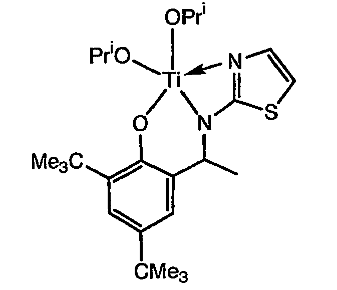

Zur Lösung des Ti(Oi-Pr)₄ (130 mg, 457 µmol) in 15 mL Et₂O wurde unter Rühren bei 0 °C die Lösung des Liganden [Lig5]H₂ (9) (115 mg, 346 µmol) zugetropft. Nach beendeter Zugabe wurde weitere 2 h bei Raumtemperatur gerührt, wonach die flüchtigen Bestandteile der Reaktionsmischung unter reduziertem Druck entfernt wurden. Der Rückstand wurde mit Pentan aufgenommen und auf -70 °C gekühlt. Der ausgefallene orange Feststoff wurde unter Kühlung abfiltriert und im Vakuum getrocknet. Ausbeute: 71% (122 mg, 246 µmol).
**¹H-NMR** (400 MHz, C₆D₆): δ = 0.81 (d, ³*J*_{HH} = 6.4 Hz, 3 H, OCH(C*H*₃)₂), 1.12 (d, ³*J*_{HH} = 6.4 Hz, 3 H, OCH(C*H*₃)₂), 1.20 (d, ³*J*_{HH} = 6.0 Hz, 3 H, OCH(C*H*₃)₂), 1.24 (d, ³*J*_{HH} = 6.0 Hz, 3 H, OCH(C*H*₃)₂), 1.33 (s, 9 H, C4-C(CH₃)₃), 1.68 (s, 9 H, C6-C(CH₃)₃), 1.83 (d, ³*J*_{HH} = 6.8 Hz, 3 H, CH(N)(C*H*₃)), 4.78 (sept, ³*J*_{HH} = 6.4 Hz, 1 H, OC*H*(CH₃)₂), 4.85 (quart, ³*J*_{HH} = 6.8 Hz, 1 H, C*H*(N)(CH₃)), 4.93 (sept, ³*J*_{HH} = 6.0 Hz, 1 H, OC*H*(CH₃)₂), 6.01 (d, ³*J*_{HH} = 4.4 Hz, 1H, NCH=CHS), 7.22 (d, ⁴*J*_{HH} = 2.4 Hz, 1 H, C3-H), 7.43 (d, ⁴*J*_{HH} = 2.4 Hz, 1 H, C5-H), 7.79 (d, ³*J*_{HH} = 4.4 Hz, H, NCH=CHS).

### 3.8 [Lig1]Hf(NMe₂)₂ (20) (Umsetzung von Verbindung (5) aus Beispiel 2.2.1)

Zur Lösung des Hf(NMe₂)₄ (46 mg, 130 µmol) in 8 mL Et₂O wurde unter Rühren bei -70 °C die Lösung des Liganden [Lig1]H₂ (**5**) (41 mg, 129 µmol) in 8 mL Et₂O zugetropft. Man ließ langsam auf Raumtemperatur erwärmen und entfernte die flüchtigen Bestandteile der Reaktionsmischung unter reduziertem Druck. Der Rückstand wurde mit 5 mL Pentan aufgenommen, 15 min bei Raumtemperatur gerührt und anschließend im Vakuum getrocknet. Ausbeute (aus NMR): 69 mg (118 µmol, 91%).
**¹H-NMR** (400 MHz, C₆D₆): δ = 1.28 (s, 9 H, C4-C(CH₃)₃), 1.33 (s, 9 H, C6-C(CH₃)₃), 1.47 (s, 9 H, C4-C(CH₃)₃), 1.71 (s, 9 H, C6-C(CH₃)₃), 2.70 (s, 6 H, N(CH₃)₂), 2.97 (s, 6 H, N(CH₃)₂), 3.18 (s, 6 H, N(CH₃)₂), 3.35 (s, 6 H, N(CH₃)₂), 3.55 (d, ²*J*_{HH} (Spinsystem 1) = 14.0 Hz, 1 H, CH₂), 3.86 (d, ²*J*_{HH} (Spinsystem 1) = 14.0 Hz, 1 H, CH₂), 4.11 (d, ²*J*_{HH} (Spinsystem 2) = 15.8 Hz, 1 H, CH₂), 5.03 (d, ²*J*_{HH} (Spinsystem 2) = 15.8 Hz, 1 H, CH₂), 5.64 (d, ³*J*_{HH} (Spinsystem 3) = 4.0 Hz, 1 H, NCH=C*H*S), 5.74 (d, ³*J*_{HH} (Spinsystem 4) = 4.4 Hz, 1 H, NCH=C*H*S), 6.68 (d, ³*J*_{HH} (Spinsystem 3) = 4.0 Hz, 1 H, NC*H*=CHS), 6.86 (s, 1 H, C3-H), 7.25 (d, ³*J*_{HH} (Spinsystem 4) = 4.0 Hz, 1 H, NC*H*=CHS), 7.33 (s, 1 H, C3-H), 7.48 (s, 1 H, C5-H), 7.49 (s, 1 H, C5-H).

### 4. Polymerisation von Ethen

Der Titankomplex, Toluol und MAO (Methylaluminoxan)-Lösung wurden im Reaktor vorgelegt. Unter Rühren wurde Ethen eingeleitet. Der Druck wurde während der Reaktion konstant gehalten. Nach beendeter Polymerisationszeit wurde die Reaktion durch Ablassen des Ethendruckes abgebrochen. Die Reaktionsmischung wurde in eine 10%-ige HCl-Methanollösung gegeben. Der dabei ausgefallene farblose Feststoff sowie das bereits während der Reaktion aus der Lösung aufgeschwemmte Produkt wurden für 2 h in der Fälllösung gerührt. Dann wurde das Polymer abfiltriert, mit Methanol und Hexan gewaschen, 2 h bei 60°C im Ölpumpenvakuum getrocknet und anschließend analysiert.

### 4.1 [Lig3]Ti(Oi-Pr)₂ (17) / MAO

- **Bedingungen:**: 2 mg (4.0 µmol) [Lig3]Ti(O*i*-Pr)₂ **(17)**
1.54 g (4.0 mmol) 7%-ige MAO-Lösung
40 mL Toluol
T = 50 °C, t = 30 min, p_{Ethen} = 4 bar
- **Ausbeute/Aktivität:**: 55 mg, 27.5 kg_{PE}/(h·mol_{Ti})

### 4.2 [Lig3]Ti(Oi-Pr)₂ (17) / i-Bu₃Al / MAO

- **Bedingungen:**: 2 mg (4.0 µmol) [Lig3]Ti(O*i*-Pr)₂ **(17)**
39 mg (197 µmol) *i*-Bu₃Al
1.54 g (4.0 mmol) 7%-ige MAO-Lösung
40 mL Toluol
T = 50 °C, t = 30 min, p_{Ethen} = 4 bar
- **Ausbeute/Aktivität:**: 64 mg, 32.0 kg_{PE}/(h·mol_{Ti})

### 4.3 {[Lig₃]H}₂Ti(Oi-Pr)₂ (18) / MAO

- **Bedingungen:**: 5 mg (6.0 µmol) {[Lig3]H}₂Ti(O*i*-Pr)₂ **(18)**
567 mg (2.1 mmol) 10%-ige MAO-Lösung
40 mL Toluol
T = 50°C, t = 30 min, p_{Ethen} = 4 bar
- **Ausbeute/Aktivität:**: 79 mg, 26.3 kg_{PE}/(h·mol_{Ti})

### 4.4 {[Lig1]H}₂TiCl₂ (15) / MAO

- **Bedingungen:**: 5 mg (6.5 µmol) {[Ligl]H}₂TiCl₂ **(15)**
883 mg (2.29 mmol) 7%-ige MAO-Lösung
40 mL Toluol
T = 25 °C, t = 30 min, p_{Ethen} = 4 bar
- **Ausbeute/Aktivität:**: 237 mg, 72.9 kg_{PE}/(h·mol_{Ti})

### 4.5 [Lig5]Ti(Oi-Pr)₂ (19) / MAO

- **Bedingungen:**: 2 mg (4.0 µmol) [Lig3]Ti(Oi-Pr)₂ **(19)**
1.54 g (4.0 mmol) 7%-ige MAO-Lösung
40 mL Toluol
T = 50 °C, t = 30 min, p_{Ethen} = 4 bar
- **Ausbeute/Aktivität:**: 136 mg, 68.0 kg_{PE}/(h·mol_{Ti})

## Patentansprüche

1. Verbindung der allgemeinen Formel Ia oder Ib 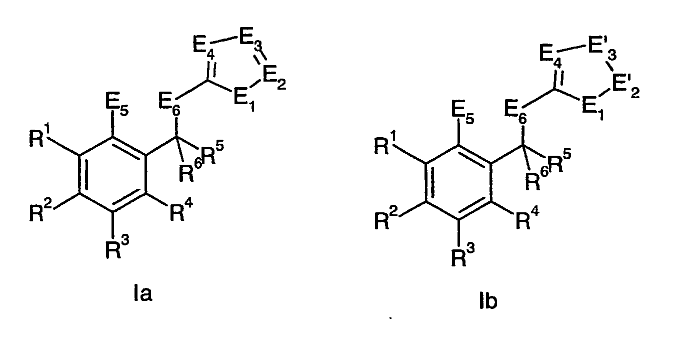 worin
in Formel Ia
E1 O, S, Se, Te, NR, CR₂, PR
E2, E3 CR, N, P
E4 N, P
E5 OH, SH, NHR oder OR', SR', NRR'
E6 NH, PH oder NR', PR'
R⁵, R⁶ Wasserstoff oder ein linearer, verzweigter oder cyclischer Alkylrest, ein Arylrest
R¹, R², R³, R⁴ Wasserstoff, ein linearer, verzweigter oder cyclischer Alkylrest, ein Arylrest, Halogen oder eine Nitrogruppe
R Wasserstoff, ein linearer, verzweigter oder cyclischer Alkylrest
R' ein linearer, verzweigter oder cyclischer Alkylrest
bedeuten,
wobei mindestens eine der Gruppen E5 oder E6 ein Wasserstoffatom enthält;
in Formel Ib
die Symbole E1, E4, E5, E6, R⁵, R⁶, R¹, R², R³, R⁴, R und R' dieselben Bedeutungen aufweisen wie in Formel Ia
und
E2' und E3' O, S, Se, Te, NR, CR₂, PR bedeuten.

2. Verbindung nach Anspruch 1, **dadurch gekennzeichnet, dass** E1 S bedeutet.

3. Verbindung nach Anspruch 1 oder 2, **dadurch gekennzeichnet, dass** E4 N bedeutet.

4. Verbindung nach einem der Ansprüche 1 bis 3, **dadurch gekennzeichnet, dass** E6 NH bedeutet.

5. Verfahren zur Herstellung einer Verbindung nach einem der Ansprüche 1 bis 4, **dadurch gekennzeichnet, dass** eine Verbindung der allgemeinen Formel IIa oder IIb mit einer Verbindung der allgemeinen Formel III zu einer Verbindung der allgemeinen Formel IVa oder IVb umgesetzt wird (Schritt a)) und die Verbindung der allgemeinen Formel IVa oder IVb anschließend zu einer Verbindung der allgemeinen Formel Ia oder Ib reduziert wird (Schritt b)):
a) 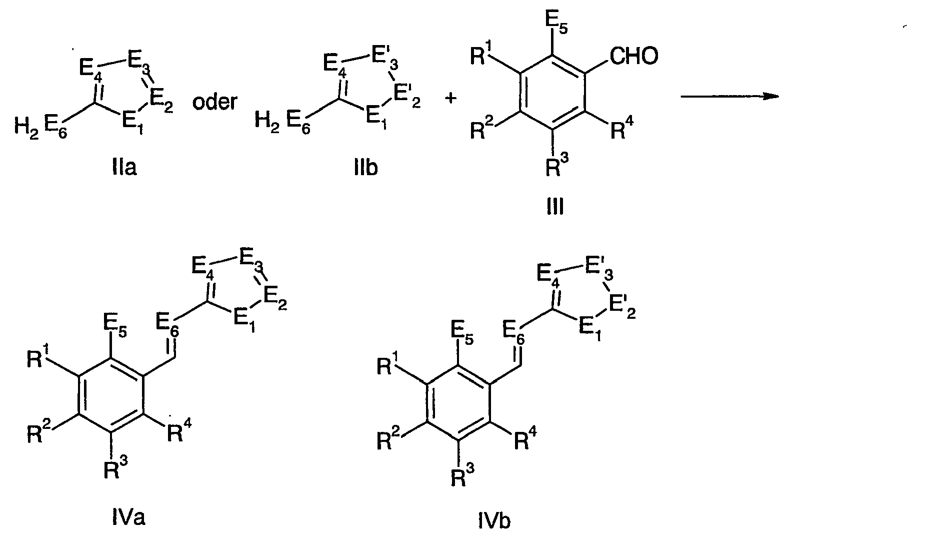
b) 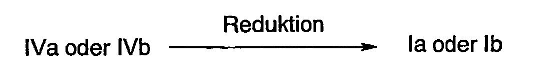
worin
E1 O, S, Se, Te, NR, CR₂, PR, bevorzugt S
E2, E3 CR, N, P
E2',E3' O, S, Se, Te, NR, CR₂, PR
E4 N, P, bevorzugt N
E5 OH, SH, NHR oder OR', SR', NRR'
E6 NH, PH, bevorzugt NH, oder NR', PR'
R⁵, R⁶ Wasserstoff oder ein linearer, verzweigter oder cyclischer Alkylrest, ein Arylrest
R¹, R², R³, R⁴ Wasserstoff, ein linearer, verzweigter oder cyclischer Alkylrest, ein Arylrest, Halogen oder eine Nitrogruppe
R Wasserstoff, ein linearer, verzweigter oder cyclischer Alkylrest
R' ein linearer, verzweigter oder cyclischer Alkylrest
bedeuten,
wobei mindestens eine der Gruppen E5 oder E6 ein Wasserstoffatom enthält.

6. Verwendung einer Verbindung nach einem der Ansprüche 1 bis 4 zur Herstellung von Metallkomplexen.

7. Metallkomplex der allgemeinen Formel V
LₓMR"_{y}Y_{z} (V)
worin
L ein von den Verbindungen gemäß einer der allgemeinen Formeln Ia oder Ib nach einem der Ansprüche 1 bis 4 abgeleiteter mono- oder dianionischer Ligand ist,
worin
- im Falle eines dianionischen Liganden -
E5 O-, S-, RN⁻, bevorzugt O-, und
E6 N, P-, bevorzugt N-,
und
bedeuten
- im Falle eines monoanionischen Liganden -
entweder
E5 O⁻, S⁻, RN⁻, bevorzugt O⁻, und
E6 NR, PR
bedeuten
oder
E5 OR, SR, NRR', und
E6 N⁻, P⁻, bevorzugt N⁻,
bedeuten,
und die weiteren Symbole E1, E2, E2', E3, E3', E4, R⁵, R⁶, R¹, R², R³, R⁴, R und R' der allgemeinen Formeln I und II dieselbe Bedeutung aufweisen wie die entsprechenden Symbole gemäß einem der Ansprüche 1 bis 4;
und - in dem Fall, dass L ein dianionischer Ligand ist -
M Ti, Zr, Hf, V, Nb, Ta, Cr, Mo, W, bevorzugt Ti, Zr, Hf,
R" Wasserstoff, ein Kohlenwasserstoffrest, bevorzugt ein linearer, verzweigter oder cyclischer Alkylrest, NR"'₂, OR"', Halogen, Acetylacetonat, bevorzugt Halogen, OR"', wobei R'" Wasserstoff oder ein linearer, verzweigter oder cyclischer Alkylrest ist,
Y eine Lewis-Base
x 1 oder 2, bevorzugt 1
y 1 bis 4, bevorzugt 2
z 0 bis 2, bevorzugt 0
bedeuten,
wobei R" und Y zu einem gemeinsamen Rest verbunden sein können und 2 x + y die Wertigkeit von M ergibt;
oder -in dem Fall, dass L ein monoanionischer Ligand ist -
M Ti, Zr, Hf, V, Nb, Ta, Cr, Mo, W, Ni, Pd, Co, Fe, Cu, Ru, Rh, bevorzugt Ti, Zr, Hf, Ni, Pd,
R" Wasserstoff, ein Kohlenwasserstoffrest, bevorzugt ein linearer, verzweigter oder cyclischer Alkylrest, NR"'₂, OR"', Halogen, Acetylacetonat, bevorzugt Halogen, OR"', wobei R"' Wasserstoff oder ein linearer, verzweigter oder cyclischer Alkylrest ist,
Y eine Lewis-Base
x 1, 2 oder 3,
y 1 bis 4,
z 0 bis 2, bevorzugt 0
bedeuten,
wobei R" und Y zu einem gemeinsamen Rest verbunden sein können und x + y die Wertigkeit von M ergibt.

8. Metallkomplex nach Anspruch 7, **dadurch gekennzeichnet, dass** der Ligand L ein dianionischer Ligand ist und M Ti, Zr oder Hf ist.

9. Metallkomplex nach Anspruch 8, **dadurch gekennzeichnet, dass** x 1, y 2 und z 0 ist.

10. Metallkomplex nach Anspruch 7, **dadurch gekennzeichnet dass** der Ligand L ein monoanionischer Ligand ist und M Ti, Zr, Hf, Ni oder Pd ist.

11. Metallkomplex nach Anspruch 10, **dadurch gekennzeichnet, dass** in dem Fall, wenn M Ti, Zr, Hf ist, x 2, y 2 und z 0 ist oder x 1, y 3 und z 0 ist und, wenn M Ni oder Pd ist, x 1, y 1 und z 0 ist.

12. Verfahren zur Herstellung eines Metallkomplexes nach einem der Ansprüche 7 bis 11, durch Deprotonierung einer Verbindung nach Anspruch 1 bis 4 mit einer Base und anschließende Umsetzung mit einer Metallverbindung, oder durch direkte Umsetzung einer Verbindung nach einem der Ansprüche 1 bis 4 mit einer Metallverbindung,
wobei die Metallverbindung ein Metall M ausgewählt aus der Gruppe bestehend aus Ti, Zr, Hf, V, Nb, Ta, Cr, Mo und W, bevorzugt Ti, Zr, Hf in dem Fall, dass L ein dianionischer Ligand ist, oder ein Metall M ausgewählt aus der Gruppe bestehend aus Ti, Zr, Hf, V, Nb, Ta, Cr, Mo, W, Ni, Pd, Co, Fe, Cu, Ru und Rh, bevorzugt Ti, Zr, Hf, Ni, Pd, in dem Fall, dass L ein monoanionischer Ligand ist, enthält.

13. Katalytisch aktive Zusammensetzung umfassend
(a) einen Metallkomplex der allgemeinen Formel V nach einem der Ansprüche 7 bis 11, als Komponente A,
(b) mindestens eine Verbindung, als Komponente B, ausgewählt aus der Gruppe bestehend aus
(b1) einer organometallischen Verbindung, als Komponente B1,
(b2) einer Organoaluminium-oxy-Verbindung, als Komponente B2, und
(b3) einer Verbindung, die mit dem Metallkomplex unter Ausbildung eines Ionenpaares reagiert, als Komponente B3.

14. Katalytisch aktive Zusammensetzung nach Anspruch 13, **dadurch gekennzeichnet, dass** zusätzlich zu den Komponenten A und B ein Trägermaterial (Komponente C) enthalten ist.

15. Verfahren zur Herstellung einer katalytisch aktiven Zusammensetzung nach Anspruch 13 oder 14, **dadurch gekennzeichnet, dass** ein Metallkomplex der allgemeinen Formel V nach einem der Ansprüche 7 bis 11 (Komponente A) mit einer Verbindung (Komponente B) ausgewählt aus der Gruppe bestehend aus
(b1) einer organometallischen Verbindung, als Komponente B1,
(b2) einer Organoaluminium-oxy-Verbindung, als Komponente B2, und
(b3) einer Verbindung, die mit dem Metallkomplex unter Ausbildung eines Ionenpaares reagiert, als Komponente B3,
und gegebenenfalls einem Trägermaterial (Komponente C) in Kontakt gebracht wird.

16. Verwendung einer katalytisch aktiven Zusammensetzung nach Anspruch 13 oder 14 zur Polymerisation oder Copolymerisation von Olefinen.

17. Verfahren zur Polymerisation oder Copolymerisation von Olefinen, wobei ein Olefin in Gegenwart einer katalytisch aktiven Zusammensetzung nach Anspruch 13 oder 14 polymerisiert wird oder mindestens zwei verschiedene Olefine in Gegenwart einer katalytisch aktiven Zusammensetzung nach Anspruch 13 oder 14 copolymerisiert werden.

## Claims

1. A compound of the general formula Ia or Ib 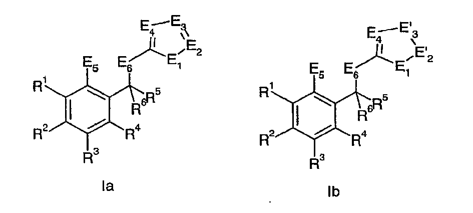 in which
in formula Ia
E1 means O, S, Se, Te, NR, CR₂, PR
E2, E3 mean CR, N, P
E4 means N, P
E5 means OH, SH, NHR or OR', SR', NRR'
E6 means NH, PH or NR', PR¹
R⁵, R⁶ mean hydrogen or a linear, branched or cyclic alkyl radical, an aryl radical
R¹, R², R³, R⁴ mean hydrogen, a linear, branched or cyclic alkyl radical, an aryl radical, halogen or a nitro group
R means hydrogen, a linear, branched or cyclic alkyl radical
R' means a linear, branched or cyclic alkyl radical,
wherein at least one of the groups E5 or E6 contains a hydrogen atom;
in formula Ib
the symbols E1, E4, E5, E6, R⁵, R⁶, R¹, R², R³, R⁴, R and
R' have the same meanings as in formula Ia and
E2' and E3' mean O, S, Se, Te, NR, CR₂, PR.

2. A compound according to claim 1, **characterised in that** E1 means S.

3. A compound according to claim 1 or claim 2, **characterised in that** E4 means N.

4. A compound according to any one of claims 1 to 3, **characterised in that** E6 means NH.

5. A method for producing a compound according to any one of claims 1 to 4, **characterised in that** a compound of the general formula IIa or IIb is reacted with a compound of the general formula III to yield a compound of the general formula IVa or IVb (step a)) and the compound of the general formula IVa or IVb is then reduced to yield a compound of the general formula Ia or Tb (step b)):
a) 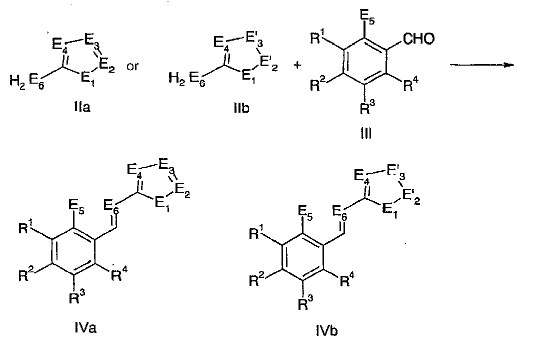
b) 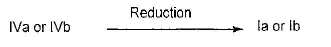
in which
E1 means O, S, Se, Te, NR, CR₂, PR, preferably S
E2, E3 mean CR, N, P
E2', E3' mean O, S, Se, Te, NR, CR₂, PR
E4 means N, P, preferably N
E5 means OH, SH, NHR or OR', SR', NRR'
E6 means NH, PH, preferably NH, or NR', PR'
R⁵, R⁶ mean hydrogen or a linear, branched or cyclic alkyl radical, an aryl radical
R¹, R², R³, R⁴ mean hydrogen, a linear, branched or cyclic alkyl radical, an aryl radical, halogen or a nitro group
R means hydrogen, a linear, branched or cyclic alkyl radical
R' means a linear, branched or cyclic alkyl radical,
wherein at least one of groups E5 or E6 contains a hydrogen atom.

6. Use of a compound according to any one of claims 1 to 4 for producing metal complexes.

7. A metal complex of the general formula V
LₓMR"_{y}Y_{z} (V)
in which
L is a mono- or dianionic ligand derived according to any one of claims 1 to 4 from the compounds according to one of the general formulae Ia or Ib, in which
- in the case of a dianionic ligand -
E5 means O⁻, S⁻, RN⁻, preferably O⁻, and
E6 means N⁻, P⁻, preferably N⁻,
and
- in the case of a monoanionic ligand -
either
E5 means O⁻, S⁻, RN⁻, preferably O⁻, and
E6 means NR, PR
or
E5 means OR, SR, NRR', and
E6 means N⁻, P⁻, preferably N⁻,
and the further symbols E1, E2, E2', E3, E3', E4, R⁵, R⁶, R¹, R², R³, R⁴, R and R' of the general formulae I and II have the same meaning as the corresponding symbols according to any one of claims 1 to 4;
and - in the event that L is a dianionic ligand -
M means Ti, Zr, Hf, V, Nb, Ta, Cr, Mo, W, preferably Ti, Zr, Hf,
R" means hydrogen, a hydrocarbon radical, preferably a linear, branched or cyclic alkyl radical, NR"'₂, OR''', halogen, acetylacetonate, preferably halogen, OR''', wherein R"' is hydrogen or a linear, branched or cyclic alkyl radical,
Y means a Lewis base
x means 1 or 2, preferably 1
y means 1 to 4, preferably 2
z means 0 to 2, preferably 0
wherein R" and Y may be joined to form a common radical and 2 x + y is the valency of M;
or - in the event that L is a monoanionic ligand -
M means Ti, Zr, Hf, V, Nb, Ta, Cr, Mo, W, Ni, Pd, Co, Fe, Cu, Ru, Rh, preferably Ti, Zr, Hf, Ni, Pd,
R" means hydrogen, a hydrocarbon radical, preferably a linear, branched or cyclic alkyl radical, NR"'₂, OR''', halogen, acetylacetonate, preferably halogen, OR''', wherein R"' is hydrogen or a linear, branched or cyclic alkyl radical,
Y means a Lewis base
x means 1, 2 or 3,
y means 1 to 4,
z means 0 to 2, preferably 0,
wherein R" and Y may be joined to form a common radical and x + y is the valency of M.

8. A metal complex according to claim 7, **characterised in that** the ligand L is a dianionic ligand and M is Ti, Zr or Hf.

9. A metal complex according to claim 8, **characterised in that** x is 1, y is 2 and z is 0.

10. A metal complex according to claim 7, **characterised in that** the ligand L is a monoanionic ligand and M is Ti, Zr, Hf, Ni or Pd.

11. A metal complex according to claim 10, **characterised in that**, if M is Ti, Zr, Hf, x is 2, y is 2 and z is 0 or x is 1, y is 3 and z is 0 and, if M is Ni or Pd, x is 1, y is 1 and z is 0.

12. A method for producing of a metal complex according to any one of claims 7 to 11 by deprotonation of a compound according to claims 1 to 4 with a base and subsequent reaction with a metal compound, or by direct reaction of a compound according to any one of claims 1 to 4 with a metal compound,
wherein the metal compound contains a metal M selected from the group consisting of Ti, Zr, Hf, V, Nb, Ta, Cr, Mo and W, preferably Ti, Zr, Hf, in the event that L is a dianionic ligand, or a metal M selected from the group consisting of Ti, Zr, Hf, V, Nb, Ta, Cr, Mo, W, Ni, Pd, Co, Fe, Cu, Ru and Rh, preferably Ti, Zr, Hf, Ni, Pd, in the event that L is a monoanionic ligand.

13. A catalytically active composition comprising
(a) a metal complex of the general formula V according to any one of claims 7 to 11 as component A,
(b) at least one compound as component B, selected from the group consisting of
(b1) an organometallic compound as component B1,
(b2) an organoaluminium oxy compound as component B2, and
(b3) a compound which reacts with the metal complex to form an ion pair as component B3.

14. A catalytically active composition according to claim 13, **characterised in that** a support material (component C) is present in addition to components A and B.

15. A method for producing a catalytically active composition according to claim 13 or claim 14, **characterised in that** a metal complex of the general formula V according to any one of claims 7 to 11 (component A) is brought into contact with a compound (component B) selected from the group consisting of
(b1) an organometallic compound as component B1,
(b2) an organoaluminium oxy compound as component B2, and
(b3) a compound which reacts with the metal complex to form an ion pair as component B3,
and optionally with a support material (component C).

16. Use of a catalytically active composition according to claim 13 or claim 14 for polymerising or copolymerising olefins.

17. A method for polymerising or copolymerising olefins,
wherein an olefin is polymerised in the presence of a catalytically active composition according to claim 13 or claim 14 or at least two different olefins are copolymerised in the presence of a catalytically active composition according to claim 13 or claim 14.

## Revendications

1. Composé de formule générale Ia ou Ib, 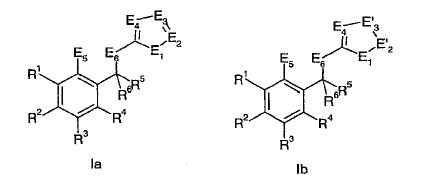 où
dans la formule la
E1 signifie O, S, Se, Te, NR, CR₂, PR
E2, E3 signifient CR, N, P
E4 signifie N, P
E5 signifie OH, SH, NHR ou OR', SR', NRR'
E6 signifie NH, PH ou NR', PR'
R⁵, R⁶ signifient hydrogène ou un radical alkyle linéaire, ramifié ou cyclique, un radical aryle
R¹, R², R³, R⁴ signifient hydrogène, un radical alkyle linéaire, ramifié ou cyclique, un radical aryle, halogène ou un groupe nitro,
R signifie hydrogène, un radical alkyle linéaire, ramifié ou cyclique,
R' signifie un radical alkyle linéaire, ramifié ou cyclique,
où au moins un des groupes E5 ou E6 contient un atome d'hydrogène ;
dans la formule Ib
les symboles E1, E4, E5, E6, R⁵, R⁶, R¹, R², R³, R⁴, R et
R' présentent les mêmes significations que dans la formule Ia
et
E2' et E3' signifient O, S, Se, Te, NR, CR₂, PR.

2. Composé selon la revendication 1, **caractérisé en ce que** E1 signifie S.

3. Composé selon la revendication 1 ou 2, **caractérisé en ce que** E4 signifie N.

4. Composé selon l'une quelconque des revendications 1 à 3, **caractérisé en ce que** E6 signifie NH.

5. Procédé pour la préparation d'un composé selon l'une quelconque des revendications 1 à 4, **caractérisé en ce qu'**on transforme un composé de formule générale IIa ou IIb avec un composé de formule générale III en un composé de formule générale IVa ou IVb (étape a)) et on réduit ensuite le composé de formule générale IVa ou IVb en un composé de formule générale Ia ou Ib (étape b)) :
a) 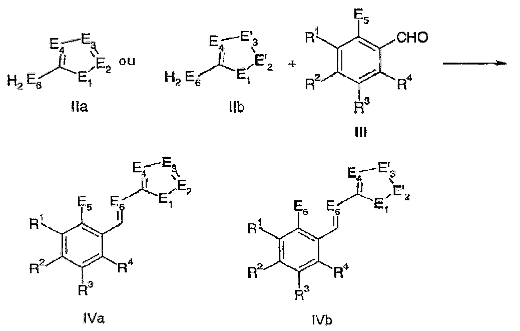
b) 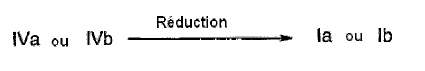
ou
E1 signifie O, S, Se, Te, NR, CR₂, PR, de préférence S,
E2, E3 signifient CR, N, P
E2',E3' signifie O, S, Se, Te, NR, CR₂, PR
E4 signifie N, P, de préférence N
E5 signifie OH, SH, NHR ou OR', SR', NRR'
E6 signifie NH, PH, de préférence NH, ou NR', PR'
R⁵, R⁶ signifient hydrogène ou un radical alkyle linéaire, ramifié ou cyclique, un radical aryle
R¹, R², R³, R⁴ signifient hydrogène, un radical alkyle linéaire, ramifié ou cyclique, un radical aryle, halogène ou un groupe nitro,
R signifie hydrogène, un radical alkyle linéaire, ramifié ou cyclique,
R' signifie un radical alkyle linéaire, ramifié ou cyclique,
où au moins un des groupes E5 ou E6 contient un atome d'hydrogène.

6. Utilisation d'un composé selon l'une quelconque des revendications 1 à 4 pour la préparation de complexes métalliques.

7. Complexe métallique de formule générale V
LₓMR"_{y}Y_{z} (V)
où
L représente un ligand monoanionique ou dianionique dérivé des composés selon l'une quelconque des formules générales Ia ou Ib selon l'une quelconque des revendications 1 à 4,
où
- dans le cas d'un ligand dianionique -
E5 signifie O⁻, S⁻, RN⁻, de préférence O⁻, et
E6 signifie N⁻, P⁻, de préférence N⁻,
et
- dans le cas d'un ligand monoanionique -
soit
E5 signifie O⁻, S⁻, RN⁻, de préférence O⁻, et
E6 signifie NR, PR ou
E5 signifie OR, SR, NRR', et
E6 signifie N⁻, P⁻, de préférence N⁻,
et les autres symboles E1, E2, E2', E3, E3', E4, R⁵, R⁶, R¹, R², R³, R⁴, R et R' des formules générales I et II présentent la même signification que les symboles correspondants selon l'une quelconque des revendications 1 à 4 ;
et - dans le cas où L représenterait un ligand dianionique -
M signifie Ti, Zr, Hf, V, Nb, Ta, Cr, Mo, W, de préférence Ti, Zr, Hf,
R" signifie hydrogène, un radical hydrocarboné, de préférence un radical alkyle linéaire, ramifié ou cyclique, NR"'₂, OR'", halogène, acétylacétonate, de préférence halogène, OR''', où R"' représente hydrogène ou un radical alkyle linéaire ou ramifié,
Y signific une base de Lewis
x vaut 1 ou 2, de préférence 1,
y vaut 1 à 4, de préférence 2
z vaut 0 à 2, de préférence 0
où R" et Y peuvent être liés en un radical commun et 2 x + y donne la valence de M ;
ou - dans le cas où L représenterait un ligand monoanionique -
M signifie Ti, Zr, Hf, V, Nb, Ta, Cr, Mo, W, Ni, Pd, Co, Fe, Cu, Ru, Rh, de préférence Ti, Zr, Hf, Ni, Pd,
R" signifie hydrogène, un radical hydrocarboné, de préférence un radical alkyle linéaire, ramifié ou cyclique, NR"'₂, OR''', halogène, acétylacétonate, de préférence halogène, OR''', où R''' représente hydrogène ou un radical alkyle linéaire, ramifié ou cyclique,
Y signifie une base de Lewis
x vaut 1, 2 ou 3
y vaut 1 à 4,
z vaut 0 à 2, de préférence 0
où R" et Y peuvent être liés en un radical commun et x + y donne la valence de M.

8. Complexe métallique selon la revendication 7, **caractérisé en ce que** le ligand L est un ligand dianionique et M représente Ti, Zr ou Hf.

9. Complexe métallique selon la revendication 8, **caractérisé en ce que** x vaut 1, y vaut 2 et z vaut 0.

10. Complexe métallique selon la revendication 7, **caractérisé en ce que** le ligand L est un ligand monoanionique et M représente Ti, Zr, Hf, Ni ou Pd.

11. Complexe métallique selon la revendication 10, **caractérisé en ce que** dans le cas où M représenterait Ti, Zr, Hf, x vaut 2, y vaut 2 et z vaut 0 ou x vaut 1, y vaut 3 et z vaut 0 et, lorsque M représente Ni ou Pd, x vaut 1, y vaut 1 et z vaut 0.

12. Procédé pour la préparation d'un complexe métallique selon l'une quelconque des revendications 7 à 11, par déprotonation d'un composé selon la revendication 1 à 4 avec une base et transformation consécutive avec un composé métallique, ou
par transformation directe d'un composé selon l'une quelconque des revendications 1 à 4 avec un composé métallique,
où le composé métallique contient un métal M choisi dans le groupe constitué par Ti, Zr, Hf, V, Nb, Ta, Cr, Mo et W, de préférence Ti, Zr, Hf dans le cas où L représenterait un ligand dianionique, ou un métal M choisi dans le groupe constitué par Ti, Zr, Hf, V, Nb, Ta, Cr, Mo, W, Ni, Pd, Co, Fe, Cu, Ru et Ph, de préférence Ti, Zr, Hf, Ni, Pd, dans le cas où L représenterait un ligand monoanionique.

13. Composition catalytiquement active, comprenant
(a) un complexe métallique de formule générale V selon l'une quelconque des revendications 7 à 11, comme composant A,
(b) au moins un composé, comme composant B, choisi dans le groupe constitué par
(b1) un composé organométallique, comme composant B1,
(b2) un composé organoaluminiumoxy, comme composant B2, et
(b3) un composé qui réagit avec le complexe métallique en formant une paire d'ions, comme composant B3.

14. Composition catalytiquement active selon la revendication 13, **caractérisée en ce qu'**elle contient, en plus des composants A et B, un matériau support (composant C).

15. Procédé pour la préparation d'une composition catalytiquement active selon la revendication 13 ou 14, **caractérisé en ce qu'**on met en contact un complexe métallique de formule générale V selon l'une quelconque des revendications 7 à 11 (composant A), avec un composé (composant B) choisi dans le groupe constitué par
(b1) un composé organométallique, comme composant B1,
(b2) un composé organoaluminiumoxy, comme composant B2, et
(b3) un composé qui réagit avec le complexe métallique en formant une paire d'ions, comme composant B3,
et le cas échéant un matériau support (composant C).

16. Utilisation d'une composition catalytiquement active selon la revendication 13 ou 14 pour la polymérisation ou la copolymérisation d'oléfines.

17. Procédé pour la polymérisation ou la copolymérisation d'oléfines, dans lequel on polymérise une oléfine en présence d'une composition catalytiquement active selon la revendication 13 ou 14 ou on copolymérise au moins deux oléfines différentes en présence d'une composition catalytiquement active selon la revendication 13 ou 14.
